# EUROPÄISCHE PATENTSCHRIFT

(11) **EP 3 160 945 B1**
(45) Veröffentlichungstag und Bekanntmachung des Hinweises auf die Patenterteilung: **31.01.2018**
(21) Anmeldenummer: 15731084.8
(22) Anmeldetag: 26.06.2015
(51) Int. Cl.: C07D 249/14, C07D 257/06, C07D 271/04, C07D 271/10, C07C 317/44, C07C 321/28, C07C 323/56, A01N 43/82, A01N 43/653, A01N 43/713

(54) **HERBIZID WIRKSAME ARYLCARBONSÄUREAMIDE**
HERBICIDAL ARYLCARBOXYLIC ACID AMIDES
AMIDE D'ACIDE CARBONIQUE D'ARYLE À ACTION HERBICIDE

(30) Priorität: 30.06.2014 EP 14174873
(43) Veröffentlichungstag der Anmeldung: 03.05.2017
(73) Patentinhaber: Bayer CropScience Aktiengesellschaft, 40789 Monheim am Rhein (DE)
(72) Erfinder: AHRENS, Hartmut, 63329 Egelsbach (DE); TIEBES, Jörg, 60431 Frankfurt (DE); WALDRAFF, Christian, 61118 Bad Vilbel (DE); DIETRICH, Hansjörg, 65835 Liederbach am Taunus (DE); GATZWEILER, Elmar, 61231 Bad Nauheim (DE); ROSINGER, Christopher Hugh, 65719 Hofheim am Taunus (DE); SCHMUTZLER, Dirk, 65795 Hattersheim (DE)
(74) Vertreter: BIP Patents
(86) Internationale Anmeldenummer: PCT/EP2015/064484
(87) Internationale Veröffentlichungsnummer: WO 2016/001073

(56) Entgegenhaltungen:
- WO-A1-2011/035874
- WO-A1-2012/028579
- WO-A1-2012/126932

## Beschreibung

Die Erfindung betrifft das technische Gebiet der Herbizide, insbesondere das der Herbizide zur selektiven Bekämpfung von Unkräutern und Ungräsern in Nutzpflanzenkulturen.

Aus WO 2012/028579 A1 sind N-(Tetrazol-5-yl)- und N-(Triazol-5-yl)arylcarbonsäureamide und ihre Verwendung als Herbizide bekannt. WO 2012/126932 A1 beschreibt N-(1,3,4-Oxadiazol-2-yl)arylcarbonsäureamide und ihre Verwendung als Herbizide. Die dort beschriebenen Wirkstoffe zeigen nicht immer eine ausreichende Wirkung gegen Schadpflanzen und/oder sie sind zum Teil nicht ausreichend verträglich mit einigen wichtigen Kulturpflanzen, wie Getreidearten, Mais und Reis.

Aufgabe der vorliegenden Erfindung ist es, alternative herbizid wirksame Wirkstoffe bereitzustellen. Diese Aufgabe wird durch die nachfolgend beschriebenen erfindungsgemäßen Arylcarbonsäureamide gelöst, die in 2-Position des Phenylrings einen Alkyl- oder Cycloalkyl-Rest, in 3-Position einen Schwefel-Rest und in 4-Position einen Cycloalkyl-Rest tragen.

Ein Gegenstand der vorliegenden Erfindung sind somit Arylcarbonsäureamide der Formel (I) oder deren Salze 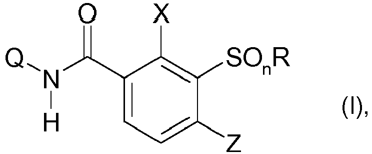 worin die Symbole und Indizes folgende Bedeutungen haben:
Q bedeutet einen Rest Q1, Q2, Q3 oder Q4, 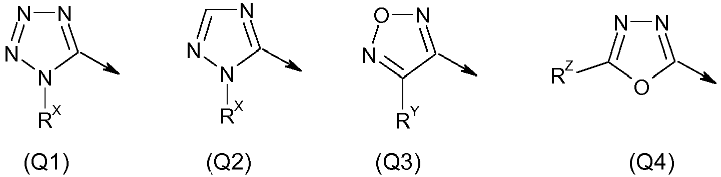
X bedeutet (C₁-C₆)-Alkyl oder (C₃-C₆)-Cycloalkyl,
Z bedeutet (C₃-C₆)-Cycloalkyl,
R bedeutet (C₁-C₆)-Alkyl, (C₃-C₆)-Cycloalkyl, (C₃-C₆)-Cycloalkyl-(C₁-C₆)-alkyl, (C₁-C₆)-Alkyl-O-(C₁-C₆)-alkyl,
R^{X} bedeutet Methyl, Ethyl, n-Propyl, Prop-2-en-1-yl, Methoxyethyl, Ethoxyethyl oder Methoxyethoxyethyl,
R^{Y} bedeutet Methyl, Ethyl, n-Propyl, Chlor oder Amino,
R^{Z} bedeutet Methyl, Ethyl, n-Propyl oder Methoxymethyl,
n bedeutet 0, 1 oder 2.

In der Formel (I) und allen nachfolgenden Formeln können Alkylreste mit mehr als zwei Kohlenstoffatomen geradkettig oder verzweigt sein. Alkylreste bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl. Analog bedeutet Alkenyl z.B. Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl. Alkinyl bedeutet z.B. Propargyl, But-2-in-1-yl, But-3-in-1-yl, 1-Methyl-but-3-in-1-yl. Die Mehrfachbindung kann sich jeweils in beliebiger Position des ungesättigten Rests befinden. Cycloalkyl bedeutet ein carbocyclisches, gesättigtes Ringsystem mit drei bis sechs C-Atomen, z.B. Cyclopropyl, Cyclobutyl, Cyclopentyl oder Cyclohexyl. Analog bedeutet Cycloalkenyl eine monocyclische Alkenylgruppe mit drei bis sechs Kohlenstoffringgliedern, z.B. Cyclopropenyl, Cyclobutenyl, Cyclopentenyl und Cyclohexenyl, wobei sich die Doppelbindung an beliebiger Position befinden kann.

Ist eine Gruppe mehrfach durch Reste substituiert, so ist darunter zu verstehen, daß diese Gruppe durch ein oder mehrere gleiche oder verschiedene der genannten Reste substituiert ist.

Die Verbindungen der allgemeinen Formel (I) können je nach Art und Verknüpfung der Substituenten als Stereoisomere vorliegen. Sind beispielsweise ein oder mehrere asymmetrisch substituierte Kohlenstoffatome vorhanden, so können Enantiomere und Diastereomere auftreten. Ebenso treten Stereoisomere auf, wenn n für 1 steht (Sulfoxide). Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft auch alle Stereoisomeren und deren Gemische, die von der allgemeinen Formel (I) umfasst, jedoch nicht spezifisch definiert sind.

Die Verbindungen der Formel (I) können Salze bilden. Salzbildung kann durch Einwirkung einer Base auf Verbindungen der Formel (I) erfolgen. Geeignete Basen sind beispielsweise organische Amine, wie Trialkylamine, Morpholin, Piperidin und Pyridin sowie Ammonium-, Alkali- oder Erdalkalimetallhydroxide, -carbonate und -hydrogencarbonate, insbesondere Natrium- und Kaliumhydroxid, Natrium- und Kaliumcarbonat und Natrium- und Kaliumhydrogencarbonat. Diese Salze sind Verbindungen, in denen der acide Wasserstoff durch ein für die Landwirtschaft geeignetes Kation ersetzt wird, beispielsweise Metallsalze, insbesondere Alkalimetallsalze oder Erdalkalimetallsalze, insbesondere Natrium- und Kaliumsalze, oder auch Ammoniumsalze, Salze mit organischen Aminen oder quartäre Ammoniumsalze, zum Beispiel mit Kationen der Formel [NR^{a}R^{b}R^{c}R^{d}]⁺, worin R^{a} bis R^{d} jeweils unabhängig voneinander einen organischen Rest, insbesondere Alkyl, Aryl, Aralkyl oder Alkylaryl darstellen. Infrage kommen auch Alkylsulfonium- und Alkylsulfoxoniumsalze, wie (C₁-C₄)-Trialkylsulfonium- und (C₁-C₄)-Trialkylsulfoxoniumsalze.

Bevorzugt sind Verbindungen der allgemeinen Formel (I), worin
Q bedeutet einen Rest Q1, Q2, Q3 oder Q4, 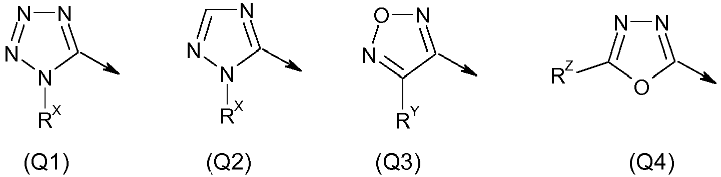
X bedeutet Methyl, Ethyl oder Cyclopropyl,
Z bedeutet Cyclopropyl oder Cyclobutyl,
R bedeutet Methyl, Ethyl, Cyclopropyl, Cyclopropylmethyl oder Methoxyethyl,
R^{X} bedeutet Methyl, Ethyl, n-Propyl, Prop-2-en-1-yl, Methoxyethyl, Ethoxyethyl oder Methoxyethoxyethyl,
R^{Y} bedeutet Methyl, Ethyl, n-Propyl, Chlor oder Amino,
R^{Z} bedeutet Methyl, Ethyl, n-Propyl oder Methoxymethyl,
n bedeutet 0, 1 oder 2.

In allen nachfolgend genannten Formeln haben die Substituenten und Symbole, sofern nicht anders definiert, dieselbe Bedeutung wie unter Formel (I) beschrieben.

Erfindungsgemäße Verbindungen, in denen Q für Q1 oder Q2 steht, sowie die diesen Amiden zugrunde liegenden Aminotetrazole und Aminotriazole können beispielsweise nach den in WO 2012/028579 angegebenen Methoden hergestellt werden.

Erfindungsgemäße Verbindungen, in denen Q für Q3 steht, sowie die diesen Amiden zugrunde liegenden Aminofurazane können beispielsweise nach den in WO 2011/035874 angegebenen Methoden hergestellt werden.

Erfindungsgemäße Verbindungen, in denen Q für Q4 steht, können beispielsweise nach den in WO 2012/126932 angegebenen Methoden hergestellt werden. Die diesen Amiden zugrunde liegenden 2-Amino-1,3,4-oxadiazole sind kommerziell verfügbar bzw. nach gängigen und in der Literatur gut bekannten Methoden synthetisch zugänglich.

Die den erfindungsgemäßen Verbindungen zugrunde liegenden Benzoesäurechloride beziehungsweise die entsprechenden Benzoesäuren können beispielsweise gemäß der in Schema 1 angegebenen Methode hergestellt werden (exemplarisch für den Rest R = Methyl angegeben). Hierzu wird 1-Brom-3-fluorbenzol einer Lithiierung unterworfen, die in die 2-Position gelenkt wird. Das Carbanion wird anschließend in den Thioether überführt. Danach wird über eine durch das Fluoratom vermittelte orthodirigierende Lithiierung mit anschließender Carboxylierung die Benzoesäure synthetisiert (Matthew D. Morrison et al., Organic Letters, 2009, vol. 11, # 5 p. 1051 - 1054; Qiuping Wang et al., Journal of Medicinal Chemistry, 2007, vol. 50, # 2 p. 199 - 210). Nach dem Aufbau der Oxazolingruppe kann das Fluoratom gegen Alkyl- oder Cycloalkylreste nucleophil ausgetauscht werden (A. I. Meyers et al., Tetrahedron Letters, 1978, 3, 223-226; A. I. Meyers et al., Tetrahedron, 1994, 50 (8), 2297-2360; T. W. Greene, P. G. M. Wuts, Protective Groups in Organic Synthesis, 2nd Edition, John Wiley & Sons, Inc. 1991, S. 265 ff.; Z. Hell et al., Tetrahedron Letters, 2002, 43, 3985-3987.). Die anschließende Oxazolinspaltung liefert die substituierte 4-Brom-3-methylthiobenzoesäure, welche als Methylester einer Kreuzkupplung unterworfen wird. Mit dem Einbau des Substituenten in der 4-Position und der anschließenden Esterverseifung ist die Synthese der Benzoesäure abgeschlossen.

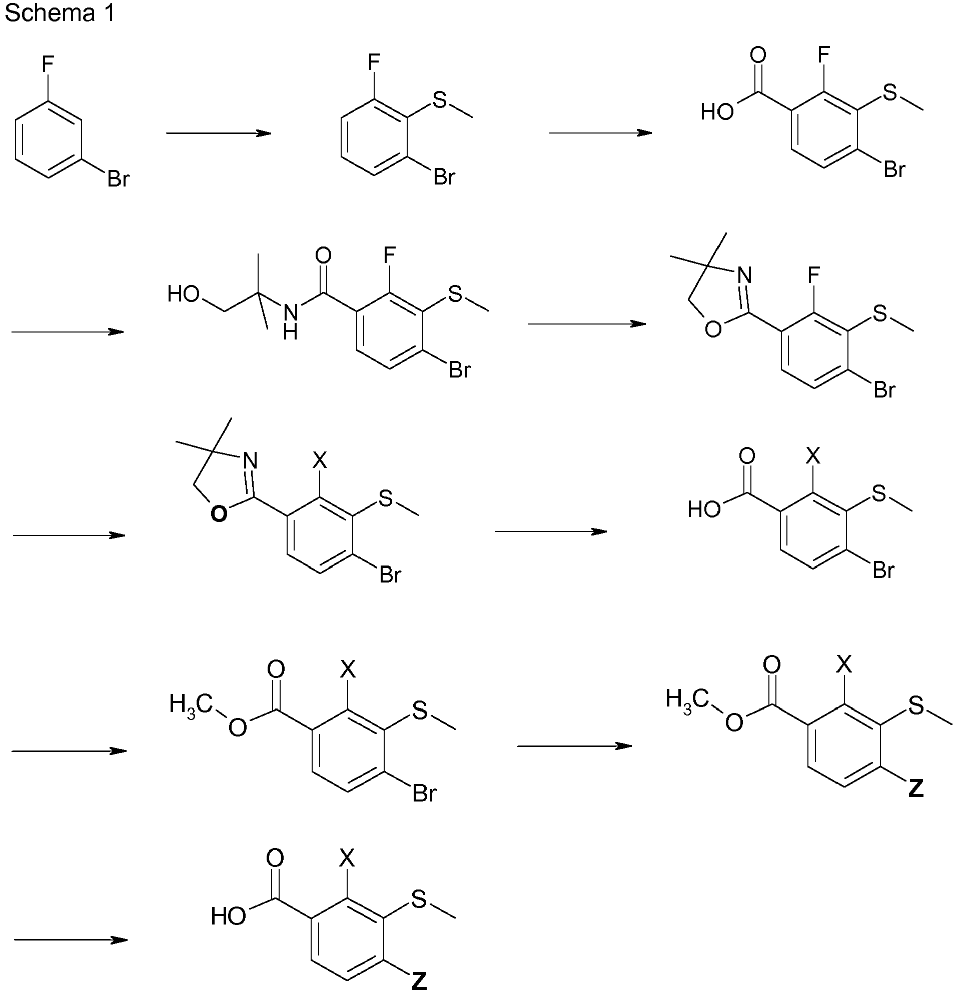

Der Thioether kann weiter zu dem entsprechenden Sulfoxid oder Sulfon oxidiert werden (Schema 2). Oxidationsmethoden, die gezielt zum Sulfoxid oder Sulfon führen, sind in der Literatur bekannt. Es bietet sich eine Anzahl an Oxidationssystemen an, beispielsweise Persäuren wie meta-Chlorperbenzoesäure, die gegebenenfalls in situ erzeugt werden (zum Beispiel Peressigsäure im System Essigsäure/Wasserstoffperoxid/Natriumwolframat(VI)) (Houben-Weyl, Methoden der Organischen Chemie, Georg Thieme Verlag Stuttgart, Bd. E 11, Erweiterungs- und Folgebände zur vierten Auflage 1985, S. 702 ff., S. 718 ff. sowie S. 1194 ff.).

Unter anderem hängt es vom Substitutionsmuster und vom Oxidationsmittel ab, an welcher Stelle der Synthesekaskade die Oxidation des Thioethers zweckmäßig ist. Eine Oxidation kann beispielsweise auf der Stufe der freien Benzoesäure oder auf der Stufe des Amids der Formel (I) mit n = 0 sinnvoll sein (Schema 2).

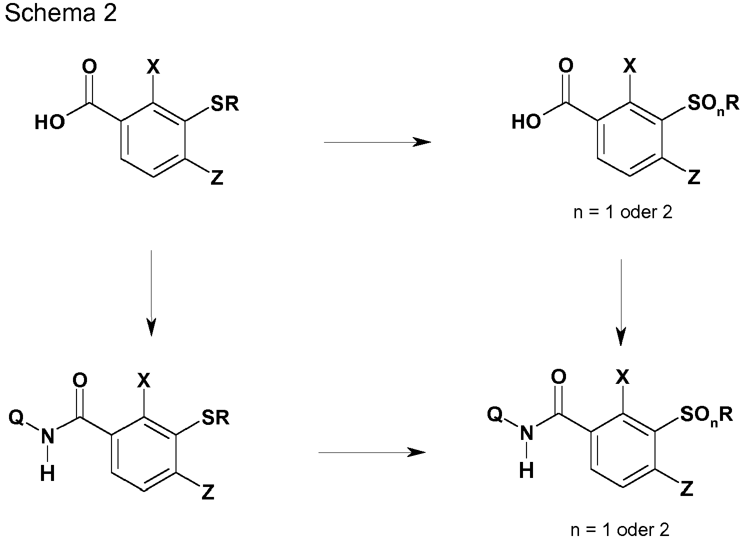

Es kann zweckmäßig sein, Reaktionsschritte in ihrer Reihenfolge zu ändern. So sind Benzoesäuren, die ein Sulfoxid tragen, nicht ohne weiteres in ihre Säurechloride zu überführen. Hier bietet sich an, zunächst auf Thioether-Stufe das Amid der Formel (I) mit n = 0 herzustellen und danach den Thioether zum Sulfoxid zu oxidieren.
Die Aufarbeitung der jeweiligen Reaktionsmischungen erfolgt in der Regel nach bekannten Verfahren, beispielsweise durch Kristallisation, wässrig-extraktive Aufarbeitung, durch chromatographische Methoden oder durch Kombination dieser Methoden.

Die bei der Herstellung der erfindungsgemäßen Verbindungen der Formel (I) als Intermediate genutzten Benzoesäuren der Formel (II) und Benzoesäurechloride der Formel (III) sind neu und ebenfalls ein Gegenstand vorliegender Erfindung.

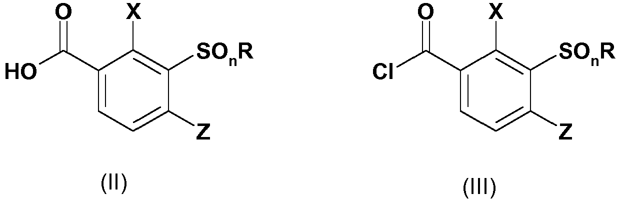

Kollektionen aus Verbindungen der Formel (I) und/oder deren Salzen, die nach den oben genannten Reaktionen synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch D. Tiebes in Combinatorial Chemistry - Synthesis, Analysis, Screening (Herausgeber Günther Jung), Verlag Wiley 1999, auf den Seiten 1 bis 34 beschrieben ist. Zur parallelisierten Reaktionsdurchführung und Aufarbeitung können eine Reihe von im Handel erhältlichen Geräten verwendet werden, beispielsweise Calypso-Reaktionsblöcke der Firma Barnstead International, Dubuque, Iowa 52004-0797, USA oder Reaktionsstationen (reaction stations) der Firma Radleys, Shirehill, Saffron Walden, Essex, CB 11 3AZ, England oder MultiPROBE Automated Workstations der Firma Perkin Elmar, Waltham, Massachusetts 02451, USA. Für die parallelisierte Aufreinigung von Verbindungen der allgemeinen Formel (I) und deren Salzen beziehungsweise von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise durch Roboter bedient werden. Derartige Automationssysteme können zum Beispiel von der Firma Caliper, Hopkinton, MA 01748, USA bezogen werden.

Die Durchführung einzelner oder mehrerer Syntheseschritte kann durch den Einsatz von Polymer-supported reagents/Scavanger-Harze unterstützt werden. In der Fachliteratur sind eine Reihe von Versuchsprotokollen beschrieben, beispielsweise in ChemFiles, Vol. 4, No. 1, Polymer-Supported Scavengers and Reagents for Solution-Phase Synthesis (Sigma-Aldrich).

Neben den hier beschriebenen Methoden kann die Herstellung von Verbindungen der allgemeinen Formel (I) und deren Salzen vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepassten Synthese an ein Syntheseharz gebunden. Festphasenunterstützte Synthesemethoden sind in der Fachliteratur hinreichend beschrieben, z.B. Barry A. Bunin in "The Combinatorial Index", Verlag Academic Press, 1998 und Combinatorial Chemistry - Synthesis, Analysis, Screening (Herausgeber Günther Jung), Verlag Wiley, 1999. Die Verwendung von Festphasen- unterstützten Synthesemethoden erlaubt eine Reihe von literaturbekannten Protokollen, die wiederum manuell oder automatisiert ausgeführt werden können. Die Reaktionen können beispielsweise mittels IRORI-Technologie in Mikroreaktoren (microreactors) der Firma Nexus Biosystems, 12140 Community Road, Poway, CA92064, USA durchgeführt werden.

Sowohl an fester als auch in flüssiger Phase kann die Durchführung einzelner oder mehrerer Syntheseschritte durch den Einsatz der Mikrowellen-Technologie unterstützt werden. In der Fachliteratur sind eine Reihe von Versuchsprotokollen beschrieben, beispielsweise in Microwaves in Organic and Medicinal Chemistry (Herausgeber C. O. Kappe und a. Stadler), Verlag Wiley, 2005.

Die Herstellung gemäß der hier beschriebenen Verfahren liefert Verbindungen der Formel (I) und deren Salze in Form von Substanzkollektionen, die Bibliotheken genannt werden. Gegenstand der vorliegenden Erfindung sind auch Bibliotheken, die mindestens zwei Verbindungen der Formel (I) und deren Salzen enthalten.

Die erfindungsgemäßen Verbindungen weisen eine ausgezeichnete herbizide Wirksamkeit gegen ein breites Spektrum wirtschaftlich wichtiger mono- und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfasst.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im Einzelnen seien beispielhaft einige Vertreter der mono- und dikotylen Unkrautflora genannt, die durch die die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria und Sorghum.

Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola und Xanthium.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Miscanthus, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, insbesondere Zea und Triticum, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüber hinaus weisen die erfindungsgemäßen Verbindungen, abhängig von ihrer jeweiligen chemischen Struktur und der ausgebrachten Aufwandmenge, hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Des Weiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z.B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt. Weitere besondere Eigenschaften können in einer Toleranz oder Resistenz gegen abiotische Stressoren z.B. Hitze, Kälte, Trockenheit, Salz und ultraviolette Strahlung liegen.

Bevorzugt ist die Anwendung der erfindungsgemäßen Verbindungen der Formel (I) oder deren Salze in wirtschaftlich bedeutenden transgenen Kulturen von Nutz-und Zierpflanzen, z.B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten.

Vorzugsweise können die Verbindungen der Formel (I) als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z.B. EP 0221044, EP 0131624). Beschrieben wurden beispielsweise in mehreren Fällen
- gentechnische Veränderungen von Kulturpflanzen zwecks Modifikation der in den Pflanzen synthetisierten Stärke (z.B. WO 92/011376 A, WO 92/014827 A, WO 91/019806 A),
- transgene Kulturpflanzen, welche gegen bestimmte Herbizide vom Typ Glufosinate (vgl. z.B. EP 0242236 A, EP 0242246 A) oder Glyphosate (WO 92/000377 A) oder der Sulfonylharnstoffe (EP 0257993 A, US 5,013,659) oder gegen Kombinationen oder Mischungen dieser Herbizide durch "gene stacking" resistent sind, wie transgenen Kulturpflanzen z. B. Mais oder Soja mit dem Handelsnamen oder der Bezeichnung Optimum™ GAT™ (Glyphosate ALS Tolerant).
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP 0142924 A, EP 0193259 A).
- transgene Kulturpflanzen mit modifizierter Fettsäurezusammensetzung (WO 91/013972 A).
- gentechnisch veränderte Kulturpflanzen mit neuen Inhalts- oder Sekundärstoffen z.B. neuen Phytoalexinen, die eine erhöhte Krankheitsresistenz verursachen (EP 0309862 A, EP 0464461 A)
- gentechnisch veränderte Pflanzen mit reduzierter Photorespiration, die höhere Erträge und höhere Stresstoleranz aufweisen (EP 0305398 A)
- transgene Kulturpflanzen, die pharmazeutisch oder diagnostisch wichtige Proteine produzieren ("molecular pharming")
- transgene Kulturpflanzen, die sich durch höhere Erträge oder bessere Qualitat auszeichnen
- transgene Kulturpflanzen die sich durch eine Kombinationen z.B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt; siehe z.B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z.B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z.B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z.B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.
Vorzugsweise können die erfindungsgemäßen Verbindungen (I) in transgenen Kulturen eingesetzt werden, welche gegen Wuchsstoffe, wie z.B. 2,4 D, Dicamba oder gegen Herbizide, die essentielle Pflanzenenzyme, z.B. Acetolactatsynthasen (ALS), EPSP Synthasen, Glutaminsynthasen (GS) oder Hydoxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, oder gegen beliebige Kombinationen dieser Wirkstoffe, resistent sind.

Besonders bevorzugt können die erfindungsgemäßen Verbindungen in transgenen Kulturpflanzen eingesetzt werden, die gegen eine Kombination von Glyphosaten und Glufosinaten, Glyphosaten und Sulfonylharnstoffen oder Imidazolinonen resistent sind. Ganz besonders bevorzugt können die erfindungsgemäßen Verbindungen in transgenen Kulturpflanzen wie z. B. Mais oder Soja mit dem Handelsnamen oder der Bezeichnung Optimum™ GAT™ (Glyphosate ALS Tolerant) eingesetzt werden.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen der Formel (I) als Herbizide zur Bekämpfung von Schadpflanzen in transgenen Kulturpflanzen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.

Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-ÖI-Emulsionen, versprühbare Lösungen, Suspensionskonzentrate (SC), Dispersionen auf Öl-oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.
Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986, Wade van Valkenburg, "Pesticide Formulations", Marcel Dekker, N.Y., 1973, K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry", 2nd Ed., J. Wiley & Sons, N.Y., C. Marsden, "Solvents Guide", 2nd Ed., Interscience, N.Y. 1963, McCutcheon's "Detergents and Emulsifiers Annual", MC Publ. Corp., Ridgewood N.J., Sisley and Wood, "Encyclopedia of Surface Active Agents", Chem. Publ. Co. Inc., N.Y. 1964, Schönfeldt, "Grenzflächenaktive Äthylenoxidaddukte", Wiss. Verlagsgesell., Stuttgart 1976, Winnacker-Küchler, "Chemische Technologie", Band 7, C. Hanser Verlag München, 4. Aufl. 1986.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London, J.E. Browning, "Agglomeration", Chemical and Engineering 1967, Seiten 147 ff, "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 1,0 kg/ha oder mehr Aktivsubstanz, vorzugsweise liegt sie jedoch zwischen 0,005 und 750 g/ha.

Die nachstehenden Beispiele erläutern die Erfindung.

### A. Chemische Beispiele

### 4-Cyclopropyl-2-methyl-3-(methylsulfinyl)-N-(1-methyl-1H-tetrazol-5-yl)benzamid (Beispiel-Nr. 1-2)

### Schritt 1: Synthese von 1-Brom-3-fluor-2-(methylsulfanyl)benzol

1028 ml einer 2.5M (2.57 mol) Lösung von n-Butyllithium in n-Hexan wurden in 1600 ml trockenem THF gelöst. Bei 0 °C wurden 400 ml (2.83 mol) Diisopropylamin zugegeben. Die Reaktionsmischung wurde 15 min bei dieser Temperatur gerührt. Anschließend wurde das Gemisch auf -78 °C abgekühlt. Bei dieser Temperatur wurden 287 ml (2.57 mol) 1-Brom-3-fluorbenzol tropfenweise zugegeben. Die Mischung wurde 1 h bei dieser Temperatur gerührt. Danach wurden 254 ml (2.82 mol) Dimethyldisulfid zugegeben. Anschließend wurde das Reaktionsgemisch auf Raumtemperatur (RT) aufgetaut. Nach der wässrigen Aufarbeitung wurde der Rückstand der organischen Phase bei 0.5 mbar einer fraktionierten Destillation unterworfen. Bei 87 °C wurden 504 g des gewünschten Produkts gewonnen.

### Schritt 2: Synthese von 4-Brom-2-fluor-3-(methylsulfanyl)benzoesäure

452 ml einer 2.5M (1.13 mol) Lösung von n-Butyllithium in n-Hexan wurden bei -78 °C tropfenweise zu einer Lösung von 176 ml (1.24 mol) Diisopropylamin in 550 ml trockenem THF gegeben. Die Lösung wurde 5 min bei dieser Temperatur und anschließend 15 min bei 0 °C gerührt. Danach wurde die Lösung wieder auf -78 °C abgekühlt. Im Anschluss wurde eine Lösung von 250 g (1.13 mol) 1-Brom-3-fluor-2-(methylsulfanyl)benzol in 150 ml trockenem THF tropfenweise zugegeben. Die Lösung wurde 1.5 h bei -78 °C gerührt. Danach wurden 298 g (6.78 mol) Kohlendioxid als Trockeneis zugegeben. Das Reaktionsgemisch wurde langsam auf RT aufgetaut. Zur Aufarbeitung wurde das Gemisch mit verdünnter Salzsäure auf pH = 1 angesäuert. Das Produkt wurde anschließend sechsmal mit Diethylether extrahiert. Die vereinigten organischen Phasen wurden mit einer gesättigten, wässrigen Kochsalzlösung gewaschen. Anschließend wurde das Produkt dreimal mit einer gesättigten, wässrigen Natriumhydrogencarbonat-Lösung extrahiert. Die vereinigten, wässrigen Extrakte wurden bei pH = 9 dreimal mit Diethylether gewaschen und danach langsam mit konzentrierter Salzsäure auf pH = 1 angesäuert. Das Produkt wurde dreimal mit Diethylether extrahiert und die vereinigten organischen Phasen wurden mit einer gesättigten, wässrigen Kochsalzlösung gewaschen. Schließlich wurden die vereinigten organischen Phasen über Magnesiumsulfat getrocknet, das Filtrat wurde vom Lösungsmittel befreit. Zur weiteren Aufreinigung wurde das Produkt aus Wasser umkristallisiert, wobei 275 g des gewünschten Produkts gewonnen wurden.

### Schritt 3: Synthese von 4-Brom-2-fluor-N-(1-hydroxy-2-methylpropan-2-yl)-3-(methylsulfanyl)benzamid

340 g (1.28 mol) 4-Brom-2-fluor-3-(methylsulfanyl)benzoesäure wurden in 1000 ml trockenem Dichlormethan mit 2 ml N,N-Dimethylformamid versetzt und anschließend auf eine Temperatur von 35 °C erwärmt. 271 ml (3.20 mol) Oxalylchlorid wurden langsam und tropfenweise bei dieser Temperatur zugegeben. Nach Abschluss der Gasentwicklung wurde das Gemisch so lange unter Rückfluss erhitzt, bis die Reaktionskontrolle kein Startmaterial mehr anzeigte. Anschließend wurde das Gemisch vom Lösungsmitel befreit. Zum Rückstand wurden 600 ml Toluol gegeben und das Gemisch wurde nochmals vom Lösungsmittel befreit. Das Säurechlorid wurde in 600 ml wasserfreiem Dichlormethan aufgenommen. Bei 5 °C - 25 °C wurde diese Lösung tropfenweise zu einer Mischung aus 305 ml (3.20 mol) 2-Amino-2-methylpropan-1-ol und 100 ml wasserfreiem Dichlormethan gegeben. Die Reaktionsmischung wurde 1.5 h bei 0 °C und danach 16 h bei Raumtemperatur gerührt. Zur Aufarbeitung wurde das Gemisch filtriert und das Filtrat wurde vom Lösungsmittel befreit. Als Rückstand wurden 330 g Produkt gewonnen, die ohne weitere Reinigung im nächsten Schritt eingesetzt wurden.

### Schritt 4: Synthese von 2-[4-Brom-2-fluor-3-(methylsulfanyl)phenyl]-4,4-dimethyl-4,5-dihydro-1,3-oxazol

330 g (0.98 mol) 4-Brom-2-fluor-N-(1-hydroxy-2-methylpropan-2-yl)-3-(methylsulfanyl)-benzamid wurden bei RT mit 384 ml (5.3 mol) Thionylchlorid versetzt. Nach Abschluss der Gasentwicklung wurde die Reaktionsmischung noch 1 h bei RT gerührt. Zur Aufarbeitung wurde das Gemisch vorsichtig in Wasser gegossen. Im Anschluss wurde die Mischung mit Diethylether extrahiert. Die wässrige Phase wurde auf 0 °C abgekühlt und mit 20 proz. Natronlauge alkalisiert. Das Gemisch wurde danach sofort und zügig mit Dichlormethan extrahiert. Die organische Phase wurde getrocknet und das Filtrat wurde vom Lösungsmittel befreit. Das Rohprodukt wurde aus Diisopropylether umkristallisiert, wobei 165 g des gewünschten Produkts isoliert wurden.

### Schritt 5: Synthese von 2-[4-Brom-2-methyl-3-(methylsulfanyl)phenyl]-4,4-dimethyl-4,5-dihydro-1,3-oxazol 45 g (141 mmol) 2-[4-Brom-2-fluor-3-(methylsulfanyl)phenyl]-4,4-dimethyl-4,5-dihydro-1,3-oxazol wurden bei RT in 440 ml trockenem Diethylether und unter Schutzgas langsam und tropfenweise mit

Methylmagnesiumiodid (frisch hergestellt aus 12.37 g (507 mmol) Magnesium und 71.24 g (501 mmol) lodmethan) versetzt. Es wurde darauf geachtet, dass die Temperatur nicht über 30 °C stieg. Danach wurde bei RT gerührt bis die Reaktionskontrolle kein Startmaterial mehr anzeigte. Zur Aufarbeitung wurde das Gemisch langsam in eine Mischung aus Eis und verdünnter Salzsäure gegossen. Im Anschluss wurde Natronlauge zugegeben bis der pH-Wert zwischen 7 und 8 lag. Die wässrige Phase wurde zweimal mit Diethylether extrahiert. Die vereinigten organischen Phasen wurden getrocknet und das Filtrat wurde vom Lösungsmittel befreit. Der Rückstand wurde aus Diisopropylether umkristallisiert, wobei 38 g des gewünschten Produkts gewonnen wurden.

### Schritt 6: Synthese von 4-Brom-2-methyl-3-(methylsulfanyl)benzoesäure

250 g (0.8 mol) 2-[4-Brom-2-methyl-3-(methylsulfanyl)phenyl]-4,4-dimethyl-4,5-dihydro-1,3-oxazol wurden mit 1300 ml 6M Salzsäure versetzt. Das Reaktionsgemisch wurde 24 h unter Rückfluss erhitzt. Zur Aufarbeitung wurde das Gemisch alkalisiert und zweimal mit Diethylether gewaschen. Die wässrige Phase wurde mit Salzsäure angesäuert. Das Produkt kristallisierte aus und das Gemisch wurde filtriert. Als Rückstand wurden 167 g des gewünschten Produkts gewonnen.

### Schritt 7: Synthese von 4-Brom-2-methyl-3-(methylsulfanyl)benzoesäuremethylester

10.1 g (38.7 mmol) 4-Brom-2-methyl-3-(methylsulfanyl)benzoesäure wurden in 100 ml Methanol mit 5 ml konzentrierter Schwefelsäure versetzt. Dann wurde 8 h unter Rückfluss erhitzt. Das Gemisch wurde auf RT abgekühlt und vom Lösungsmittel befreit. Der Rückstand wurde in Wasser aufgenommen und im Eisbad abgekühlt. Das Gemisch wurde filtriert und der Rückstand wurde mit gesättigter wässriger Natriumhydrogencarbonatlösung gewaschen. Der Rückstand wurde getrocknet, wobei 9.82 g des gewünschetn Produkts gewonnen wurden.

### Schritt 8: Synthese von 4-Cyclopropyl-2-methyl-3-(methylsulfanyl)benzoesäure (Beispiel-Nr. 9-1)

Unter Schutzgas wurden 1.94 g (80.0 mmol) Magnesiumspäne mit 5 ml trockenem Tetrahydrofuran versetzt. Anschließend wurde eine kleine Menge der insgesamt 9.67 g (80.0 mmol) Cyclopropylbromid sowie eine katalytische Menge Iod zugegeben um die Grignard-Reaktion zu initiieren. Nach dem Start der Grignard-Reaktion wurde das restliche Cyclopropylbromid, das vorher mit 145 ml trockenem Tetrahydrofuran verdünnt worden war, langsam zugetropft. Zur Vervollständigung der Reaktion wurde das Gemisch danach noch 15 min unter Rückfluss erhitzt. Der Inhalt wurde auf RT abgekühlt, danach wurden 800 mg (18.9 mmol) Lithiumchlorid zugegeben. Nachdem das Lithiumchlorid sich aufgelöst hatte wurde die Mischung auf -20 °C abgekühlt. Anschließend wurden 114 ml (0.7M; 79.8 mmol) einer Lösung von Zinkchlorid in trockenem Tetrahydrofuran langsam zugetropft. Die Mischung wurde noch 15 min bei dieser Temperatur gerührt. Im Anschluss wurde der Inhalt auf RT aufgetaut und zur Entfernung von Sauerstoff wurde am Reaktionskolben wiederholt Vakuum angelegt und mit Argon belüftet. Zu diesem Gemisch wurde eine Lösung von 10.0 g (36.3 mmol) 4-Brom-2-methyl-3-(methylsulfanyl)benzoesäuremethylester und 2.35 g (2.04 mmol) Tetrakis(triphenylphosphin)palladium(0) in 200 ml trockenem Tetrahydrofuran (angesetzt in der Weise, dass zuerst der substituierte Benzoesäureester gelöst wurde, am Behälter zur Entfernung von Sauerstoff wiederholt Vakuum angelegt und mit Argon belüftet wurde, im Anschluss der Palladium-Katalysator zugegeben wurde und schließlich am Behälter nochmals zur Entfernung von Sauerstoff wiederholt Vakuum angelegt und mit Argon belüftet wurde) zugegeben. Die Reaktionsmischung wurde 3 h unter Rückfluss erhitzt und anschließend auf RT abgekühlt. Zur Vervollständigung der Reaktion wurde nochmals die halbe der oben genannten Menge an Grignard-Reagenz hergestellt und entsprechend mit der Zinkchloridlösung umgesetzt. Nach der wie oben beschriebenen Sauerstoffentfernung wurde diese Menge an Kupplungsreagenz dem Kreuzkupplungsgemisch zugegeben. Zusätzlich wurde noch die halbe der oben genannten Menge an Palladiumkatalysator zugegeben. Die Reaktionsmischung wurde wieder unter Rückfluss erhitzt bis nach
2 h kein Startmaterial mehr detektierbar war. Zur Aufarbeitung wurde der Inhalt auf RT abgekühlt und mit 500 ml einer gesättigten, wässrigen Ammoniumchloridlösung versetzt. Das Gemisch wurde zweimal mit Diethylether extrahiert. Die vereinigten organischen Phasen wurden getrocknet, filtriert und schließlich so vorsichtig vom Lösungsmittel befreit, dass kein Produkt mit überging. Der Rückstand wurde in 100 ml Methanol gelöst und mit 10 ml 20 proz. Natronlauge versetzt. Das Gemisch wurde 16 h bei RT und anschließend 3 h bei 60 °C gerührt. Danach wurde das Gemisch unter Rückfluss erhitzt bis die Reaktionskontrolle kein Startmaterial mehr anzeigte. Das Gemisch wurde eingeengt und der Rückstand wurde in Wasser aufgenommen. Die Mischung wurde mit Dichlormethan gewaschen und die wässrige Phase wurde über Celite filtriert. Das Filtrat wurde mit 6M Salzsäure angesäuert und dreimal mit Dichlormethan extrahiert. Die vereinigten organischen Phasen wurden getrocknet und das Filtrat wurde etwa auf die Hälfte des Volumens eingeengt. Der Rückstand wurde dreimal mit 10 proz. Natronlauge extrahiert. Die vereinigten wässrigen Phasen wurden mit 6M Salzsäure angesäuert und im Eisbad gerührt, wobei das Produkt ausfiel. Das Gemisch wurde filtriert und getrocknet. Es wurden 7.31 g des gewünschten Produkts gewonnen.

### Schritt 9: Synthese von 4-Cyclopropyl-2-methyl-3-(methylsulfanyl)-N-(1-methyl-1H-tetrazol-5-yl)benzamid (Beispiel-Nr. 1-1)

330 mg (1.48 mmol) 4-Cyclopropyl-2-methyl-3-(methylsulfanyl)benzoesäure und 191 mg (98 % Reinheit; 1.89 mmol) 5-Amino-1-methyl-1H-tetrazol wurden in 10 ml Pyridin mit 264 mg (2.08 mmol) Oxalsäuredichlorid versetzt. Das Gemisch wurde 15 min bei RT und dann 2 h bei 50 °C gerührt. Zur Vervollständigung der Umsetzung wurde das Gemisch auf RT abgekühlt und nochmals ein Viertel der oben angegebenen Menge Oxalsäuredichlorid zugegeben. Das Gemisch wurde nochmals 2 h bei 50 °C gerührt. Zur Aufarbeitung wurde das Gemisch vom Lösungsmittel befreit.

Der Rückstand wurde in Dichlormethan und einer gesättigten wässrigen Natriumhydrogencarbonatlösung aufgenommen und gerührt. Nach der Phasentrennung wurde die organische Phase vom Lösungs-mittel befreit. Der Rückstand wurde in Toluol aufgenommen und die Mischung wurde nochmals eingeengt. Der Rückstand wurde chromatographisch gereinigt, wobei 320 mg des gewünschten Produkts isoliert wurden.

### Schritt 10: Synthese von 4-Cyclopropyl-2-methyl-3-(methylsulfinyl)-N-(1-methyl-1H-tetrazol-5-yl)benzamid (Beispiel-Nr. 1-2)

107 mg (0.35 mmol) 4-Cyclopropyl-2-methyl-3-(methylsulfanyl)-N-(1-methyl-1H-tetrazol-5-yl)benzamid wurden bei RT in 10 ml Eisessig mit 34.3 mg (35 proz.; 0.35 mmol) einer wässrigen Wasserstoffperoxidlösung versetzt. Die Reaktionsmischung wurde bei 60 °C gerührt bis die Reaktionskontrolle kein Startmaterial mehr anzeigte. Zur Aufarbeitung wurde festes Natriummetabisulfit zugegeben bis keine Peroxide mehr detektierbar waren. Das Gemisch wurde vom Lösungsmittel befreit und der Rückstand wurde in wenig Wasser aufgenommen. Nach Zugabe von 1M Salzsäure wurde das

Gemisch 10 min gerührt und anschließend filtriert. Der Rückstand wurde getrocknet und ergab 80.4 mg des gewünschten Produkts.

Die in nachfolgenden Tabellen aufgeführten Verbindungen sind ganz besonders bevorzugt.

Die verwendeten Abkürzungen bedeuten:
Me = Methyl Et = Ethyl Pr = Propyl c-Pr = cyclo-Propyl

**Tabelle 1: Erfindungsgemäße Verbindungen der allgemeinen Formel (I), worin Q für Q1 und R^{x} für eine Methylgruppe stehen**

| | | | | |
|---|---|---|---|---|
| 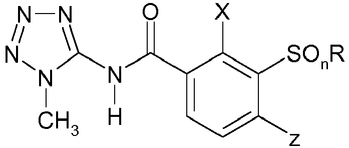 | | | | |

| Nr. | X | n | R | Z |
|---|---|---|---|---|
| 1-1 | Me | 0 | Me | c-Pr |
| 1-2 | Me | 1 | Me | c-Pr |
| 1-3 | Me | 2 | Me | c-Pr |
| 1-4 | Me | 0 | Et | c-Pr |
| 1-5 | Me | 1 | Et | c-Pr |
| 1-6 | Me | 2 | Et | c-Pr |
| 1-7 | Me | 0 | c-Pr | c-Pr |
| 1-8 | Me | 1 | c-Pr | c-Pr |
| 1-9 | Me | 2 | c-Pr | c-Pr |
| 1-10 | Me | 0 | CH₂c-Pr | c-Pr |
| 1-11 | Me | 1 | CH₂c-Pr | c-Pr |
| 1-12 | Me | 2 | CH₂c-Pr | c-Pr |
| 1-13 | Me | 0 | CH₂CH₂OMe | c-Pr |
| 1-14 | Me | 1 | CH₂CH₂OMe | c-Pr |
| 1-15 | Me | 2 | CH₂CH₂OMe | c-Pr |
| 1-16 | Me | 0 | Me | c-Bu |
| 1-17 | Me | 1 | Me | c-Bu |
| 1-18 | Me | 2 | Me | c-Bu |
| 1-19 | Me | 0 | Et | c-Bu |
| 1-20 | Me | 1 | Et | c-Bu |
| 1-21 | Me | 2 | Et | c-Bu |
| 1-22 | Me | 0 | c-Pr | c-Bu |
| 1-23 | Me | 1 | c-Pr | c-Bu |
| 1-24 | Me | 2 | c-Pr | c-Bu |
| 1-25 | Me | 0 | CH₂c-Pr | c-Bu |
| 1-26 | Me | 1 | CH₂c-Pr | c-Bu |
| 1-27 | Me | 2 | CH₂c-Pr | c-Bu |
| 1-28 | Me | 0 | CH₂CH₂OMe | c-Bu |
| 1-29 | Me | 1 | CH₂CH₂OMe | c-Bu |
| 1-30 | Me | 2 | CH₂CH₂OMe | c-Bu |
| 1-31 | Et | 0 | Me | c-Pr |
| 1-32 | Et | 1 | Me | c-Pr |
| 1-33 | Et | 2 | Me | c-Pr |
| 1-34 | Et | 0 | Et | c-Pr |
| 1-35 | Et | 1 | Et | c-Pr |
| 1-36 | Et | 2 | Et | c-Pr |
| 1-37 | Et | 0 | c-Pr | c-Pr |
| 1-38 | Et | 1 | c-Pr | c-Pr |
| 1-39 | Et | 2 | c-Pr | c-Pr |
| 1-40 | Et | 0 | CH₂c-Pr | c-Pr |
| 1-41 | Et | 1 | CH₂c-Pr | c-Pr |
| 1-42 | Et | 2 | CH₂c-Pr | c-Pr |
| 1-43 | Et | 0 | CH₂CH₂OMe | c-Pr |
| 1-44 | Et | 1 | CH₂CH₂OMe | c-Pr |
| 1-45 | Et | 2 | CH₂CH₂OMe | c-Pr |
| 1-46 | Et | 0 | Me | c-Bu |
| 1-47 | Et | 1 | Me | c-Bu |
| 1-48 | Et | 2 | Me | c-Bu |
| 1-49 | Et | 0 | Et | c-Bu |
| 1-50 | Et | 1 | Et | c-Bu |
| 1-51 | Et | 2 | Et | c-Bu |
| 1-52 | Et | 0 | c-Pr | c-Bu |
| 1-53 | Et | 1 | c-Pr | c-Bu |
| 1-54 | Et | 2 | c-Pr | c-Bu |
| 1-55 | Et | 0 | CH₂c-Pr | c-Bu |
| 1-56 | Et | 1 | CH₂c-Pr | c-Bu |
| 1-57 | Et | 2 | CH₂c-Pr | c-Bu |
| 1-58 | Et | 0 | CH₂CH₂OMe | c-Bu |
| 1-59 | Et | 1 | CH₂CH₂OMe | c-Bu |
| 1-60 | Et | 2 | CH₂CH₂OMe | c-Bu |
| 1-61 | c-Pr | 0 | Me | c-Pr |
| 1-62 | c-Pr | 1 | Me | c-Pr |
| 1-63 | c-Pr | 2 | Me | c-Pr |
| 1-64 | c-Pr | 0 | Et | c-Pr |
| 1-65 | c-Pr | 1 | Et | c-Pr |
| 1-66 | c-Pr | 2 | Et | c-Pr |
| 1-67 | c-Pr | 0 | c-Pr | c-Pr |
| 1-68 | c-Pr | 1 | c-Pr | c-Pr |
| 1-69 | c-Pr | 2 | c-Pr | c-Pr |
| 1-70 | c-Pr | 0 | CH₂c-Pr | c-Pr |
| 1-71 | c-Pr | 1 | CH₂c-Pr | c-Pr |
| 1-72 | c-Pr | 2 | CH₂c-Pr | c-Pr |
| 1-73 | c-Pr | 0 | CH₂CH₂OMe | c-Pr |
| 1-74 | c-Pr | 1 | CH₂CH₂OMe | c-Pr |
| 1-75 | c-Pr | 2 | CH₂CH₂OMe | c-Pr |
| 1-76 | c-Pr | 0 | Me | c-Bu |
| 1-77 | c-Pr | 1 | Me | c-Bu |
| 1-78 | c-Pr | 2 | Me | c-Bu |
| 1-79 | c-Pr | 0 | Et | c-Bu |
| 1-80 | c-Pr | 1 | Et | c-Bu |
| 1-81 | c-Pr | 2 | Et | c-Bu |
| 1-82 | c-Pr | 0 | c-Pr | c-Bu |
| 1-83 | c-Pr | 1 | c-Pr | c-Bu |
| 1-84 | c-Pr | 2 | c-Pr | c-Bu |
| 1-85 | c-Pr | 0 | CH₂c-Pr | c-Bu |
| 1-86 | c-Pr | 1 | CH₂c-Pr | c-Bu |
| 1-87 | c-Pr | 2 | CH₂c-Pr | c-Bu |
| 1-88 | c-Pr | 0 | CH₂CH₂OMe | c-Bu |
| 1-89 | c-Pr | 1 | CH₂CH₂OMe | c-Bu |
| 1-90 | c-Pr | 2 | CH₂CH₂OMe | c-Bu |

**Tabelle 2: Erfindungsgemäße Verbindungen der allgemeinen Formel (I), worin Q für Q1 und R^{x} für eine Ethylgruppe stehen s**

| | | | | |
|---|---|---|---|---|
| 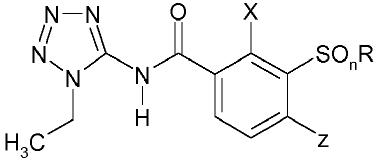 | | | | |

| Nr. | X | n | R | Z |
|---|---|---|---|---|
| 2-1 | Me | 0 | Me | c-Pr |
| 2-2 | Me | 1 | Me | c-Pr |
| 2-3 | Me | 2 | Me | c-Pr |
| 2-4 | Me | 0 | Et | c-Pr |
| 2-5 | Me | 1 | Et | c-Pr |
| 2-6 | Me | 2 | Et | c-Pr |
| 2-7 | Me | 0 | c-Pr | c-Pr |
| 2-8 | Me | 1 | c-Pr | c-Pr |
| 2-9 | Me | 2 | c-Pr | c-Pr |
| 2-10 | Me | 0 | CH₂c-Pr | c-Pr |
| 2-11 | Me | 1 | CH₂c-Pr | c-Pr |
| 2-12 | Me | 2 | CH₂c-Pr | c-Pr |
| 2-13 | Me | 0 | CH₂CH₂OMe | c-Pr |
| 2-14 | Me | 1 | CH₂CH₂OMe | c-Pr |
| 2-15 | Me | 2 | CH₂CH₂OMe | c-Pr |
| 2-16 | Me | 0 | Me | c-Bu |
| 2-17 | Me | 1 | Me | c-Bu |
| 2-18 | Me | 2 | Me | c-Bu |
| 2-19 | Me | 0 | Et | c-Bu |
| 2-20 | Me | 1 | Et | c-Bu |
| 2-21 | Me | 2 | Et | c-Bu |
| 2-22 | Me | 0 | c-Pr | c-Bu |
| 2-23 | Me | 1 | c-Pr | c-Bu |
| 2-24 | Me | 2 | c-Pr | c-Bu |
| 2-25 | Me | 0 | CH₂c-Pr | c-Bu |
| 2-26 | Me | 1 | CH₂c-Pr | c-Bu |
| 2-27 | Me | 2 | CH₂c-Pr | c-Bu |
| 2-28 | Me | 0 | CH₂CH₂OMe | c-Bu |
| 2-29 | Me | 1 | CH₂CH₂OMe | c-Bu |
| 2-30 | Me | 2 | CH₂CH₂OMe | c-Bu |
| 2-31 | Et | 0 | Me | c-Pr |
| 2-32 | Et | 1 | Me | c-Pr |
| 2-33 | Et | 2 | Me | c-Pr |
| 2-34 | Et | 0 | Et | c-Pr |
| 2-35 | Et | 1 | Et | c-Pr |
| 2-36 | Et | 2 | Et | c-Pr |
| 2-37 | Et | 0 | c-Pr | c-Pr |
| 2-38 | Et | 1 | c-Pr | c-Pr |
| 2-39 | Et | 2 | c-Pr | c-Pr |
| 2-40 | Et | 0 | CH₂c-Pr | c-Pr |
| 2-41 | Et | 1 | CH₂c-Pr | c-Pr |
| 2-42 | Et | 2 | CH₂c-Pr | c-Pr |
| 2-43 | Et | 0 | CH₂CH₂OMe | c-Pr |
| 2-44 | Et | 1 | CH₂CH₂OMe | c-Pr |
| 2-45 | Et | 2 | CH₂CH₂OMe | c-Pr |
| 2-46 | Et | 0 | Me | c-Bu |
| 2-47 | Et | 1 | Me | c-Bu |
| 2-48 | Et | 2 | Me | c-Bu |
| 2-49 | Et | 0 | Et | c-Bu |
| 2-50 | Et | 1 | Et | c-Bu |
| 2-51 | Et | 2 | Et | c-Bu |
| 2-52 | Et | 0 | c-Pr | c-Bu |
| 2-53 | Et | 1 | c-Pr | c-Bu |
| 2-54 | Et | 2 | c-Pr | c-Bu |
| 2-55 | Et | 0 | CH₂c-Pr | c-Bu |
| 2-56 | Et | 1 | CH₂c-Pr | c-Bu |
| 2-57 | Et | 2 | CH₂c-Pr | c-Bu |
| 2-58 | Et | 0 | CH₂CH₂OMe | c-Bu |
| 2-59 | Et | 1 | CH₂CH₂OMe | c-Bu |
| 2-60 | Et | 2 | CH₂CH₂OMe | c-Bu |
| 2-61 | c-Pr | 0 | Me | c-Pr |
| 2-62 | c-Pr | 1 | Me | c-Pr |
| 2-63 | c-Pr | 2 | Me | c-Pr |
| 2-64 | c-Pr | 0 | Et | c-Pr |
| 2-65 | c-Pr | 1 | Et | c-Pr |
| 2-66 | c-Pr | 2 | Et | c-Pr |
| 2-67 | c-Pr | 0 | c-Pr | c-Pr |
| 2-68 | c-Pr | 1 | c-Pr | c-Pr |
| 2-69 | c-Pr | 2 | c-Pr | c-Pr |
| 2-70 | c-Pr | 0 | CH₂c-Pr | c-Pr |
| 2-71 | c-Pr | 1 | CH₂c-Pr | c-Pr |
| 2-72 | c-Pr | 2 | CH₂c-Pr | c-Pr |
| 2-73 | c-Pr | 0 | CH₂CH₂OMe | c-Pr |
| 2-74 | c-Pr | 1 | CH₂CH₂OMe | c-Pr |
| 2-75 | c-Pr | 2 | CH₂CH₂OMe | c-Pr |
| 2-76 | c-Pr | 0 | Me | c-Bu |
| 2-77 | c-Pr | 1 | Me | c-Bu |
| 2-78 | c-Pr | 2 | Me | c-Bu |
| 2-79 | c-Pr | 0 | Et | c-Bu |
| 2-80 | c-Pr | 1 | Et | c-Bu |
| 2-81 | c-Pr | 2 | Et | c-Bu |
| 2-82 | c-Pr | 0 | c-Pr | c-Bu |
| 2-83 | c-Pr | 1 | c-Pr | c-Bu |
| 2-84 | c-Pr | 2 | c-Pr | c-Bu |
| 2-85 | c-Pr | 0 | CH₂c-Pr | c-Bu |
| 2-86 | c-Pr | 1 | CH₂c-Pr | c-Bu |
| 2-87 | c-Pr | 2 | CH₂c-Pr | c-Bu |
| 2-88 | c-Pr | 0 | CH₂CH₂OMe | c-Bu |
| 2-89 | c-Pr | 1 | CH₂CH₂OMe | c-Bu |
| 2-90 | c-Pr | 2 | CH₂CH₂OMe | c-Bu |

**Tabelle 3: Erfindungsgemäße Verbindungen der allgemeinen Formel (I), worin Q für Q1 und R^{x} für eine n-Propylgruppe stehen**

| | | | | |
|---|---|---|---|---|
| 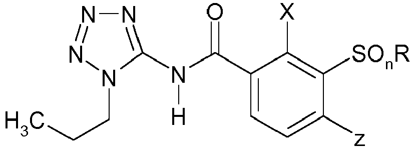 | | | | |

| Nr. | X | n | R | Z |
|---|---|---|---|---|
| 3-1 | Me | 0 | Me | c-Pr |
| 3-2 | Me | 1 | Me | c-Pr |
| 3-3 | Me | 2 | Me | c-Pr |
| 3-4 | Me | 0 | Et | c-Pr |
| 3-5 | Me | 1 | Et | c-Pr |
| 3-6 | Me | 2 | Et | c-Pr |
| 3-7 | Me | 0 | c-Pr | c-Pr |
| 3-8 | Me | 1 | c-Pr | c-Pr |
| 3-9 | Me | 2 | c-Pr | c-Pr |
| 3-10 | Me | 0 | CH₂c-Pr | c-Pr |
| 3-11 | Me | 1 | CH₂c-Pr | c-Pr |
| 3-12 | Me | 2 | CH₂c-Pr | c-Pr |
| 3-13 | Me | 0 | CH₂CH₂OMe | c-Pr |
| 3-14 | Me | 1 | CH₂CH₂OMe | c-Pr |
| 3-15 | Me | 2 | CH₂CH₂OMe | c-Pr |
| 3-16 | Me | 0 | Me | c-Bu |
| 3-17 | Me | 1 | Me | c-Bu |
| 3-18 | Me | 2 | Me | c-Bu |
| 3-19 | Me | 0 | Et | c-Bu |
| 3-20 | Me | 1 | Et | c-Bu |
| 3-21 | Me | 2 | Et | c-Bu |
| 3-22 | Me | 0 | c-Pr | c-Bu |
| 3-23 | Me | 1 | c-Pr | c-Bu |
| 3-24 | Me | 2 | c-Pr | c-Bu |
| 3-25 | Me | 0 | CH₂c-Pr | c-Bu |
| 3-26 | Me | 1 | CH₂c-Pr | c-Bu |
| 3-27 | Me | 2 | CH₂c-Pr | c-Bu |
| 3-28 | Me | 0 | CH₂CH₂OMe | c-Bu |
| 3-29 | Me | 1 | CH₂CH₂OMe | c-Bu |
| 3-30 | Me | 2 | CH₂CH₂OMe | c-Bu |
| 3-31 | Et | 0 | Me | c-Pr |
| 3-32 | Et | 1 | Me | c-Pr |
| 3-33 | Et | 2 | Me | c-Pr |
| 3-34 | Et | 0 | Et | c-Pr |
| 3-35 | Et | 1 | Et | c-Pr |
| 3-36 | Et | 2 | Et | c-Pr |
| 3-37 | Et | 0 | c-Pr | c-Pr |
| 3-38 | Et | 1 | c-Pr | c-Pr |
| 3-39 | Et | 2 | c-Pr | c-Pr |
| 3-40 | Et | 0 | CH₂c-Pr | c-Pr |
| 3-41 | Et | 1 | CH₂c-Pr | c-Pr |
| 3-42 | Et | 2 | CH₂c-Pr | c-Pr |
| 3-43 | Et | 0 | CH₂CH₂OMe | c-Pr |
| 3-44 | Et | 1 | CH₂CH₂OMe | c-Pr |
| 3-45 | Et | 2 | CH₂CH₂OMe | c-Pr |
| 3-46 | Et | 0 | Me | c-Bu |
| 3-47 | Et | 1 | Me | c-Bu |
| 3-48 | Et | 2 | Me | c-Bu |
| 3-49 | Et | 0 | Et | c-Bu |
| 3-50 | Et | 1 | Et | c-Bu |
| 3-51 | Et | 2 | Et | c-Bu |
| 3-52 | Et | 0 | c-Pr | c-Bu |
| 3-53 | Et | 1 | c-Pr | c-Bu |
| 3-54 | Et | 2 | c-Pr | c-Bu |
| 3-55 | Et | 0 | CH₂c-Pr | c-Bu |
| 3-56 | Et | 1 | CH₂c-Pr | c-Bu |
| 3-57 | Et | 2 | CH₂c-Pr | c-Bu |
| 3-58 | Et | 0 | CH₂CH₂OMe | c-Bu |
| 3-59 | Et | 1 | CH₂CH₂OMe | c-Bu |
| 3-60 | Et | 2 | CH₂CH₂OMe | c-Bu |
| 3-61 | c-Pr | 0 | Me | c-Pr |
| 3-62 | c-Pr | 1 | Me | c-Pr |
| 3-63 | c-Pr | 2 | Me | c-Pr |
| 3-64 | c-Pr | 0 | Et | c-Pr |
| 3-65 | c-Pr | 1 | Et | c-Pr |
| 3-66 | c-Pr | 2 | Et | c-Pr |
| 3-67 | c-Pr | 0 | c-Pr | c-Pr |
| 3-68 | c-Pr | 1 | c-Pr | c-Pr |
| 3-69 | c-Pr | 2 | c-Pr | c-Pr |
| 3-70 | c-Pr | 0 | CH₂c-Pr | c-Pr |
| 3-71 | c-Pr | 1 | CH₂c-Pr | c-Pr |
| 3-72 | c-Pr | 2 | CH₂c-Pr | c-Pr |
| 3-73 | c-Pr | 0 | CH₂CH₂OMe | c-Pr |
| 3-74 | c-Pr | 1 | CH₂CH₂OMe | c-Pr |
| 3-75 | c-Pr | 2 | CH₂CH₂OMe | c-Pr |
| 3-76 | c-Pr | 0 | Me | c-Bu |
| 3-77 | c-Pr | 1 | Me | c-Bu |
| 3-78 | c-Pr | 2 | Me | c-Bu |
| 3-79 | c-Pr | 0 | Et | c-Bu |
| 3-80 | c-Pr | 1 | Et | c-Bu |
| 3-81 | c-Pr | 2 | Et | c-Bu |
| 3-82 | c-Pr | 0 | c-Pr | c-Bu |
| | c-Pr | 1 | c-Pr | c-Bu |
| 3-83 | c-Pr | 2 | c-Pr | c-Bu |
| 3-84 | c-Pr | 0 | CH₂c-Pr | c-Bu |
| 3-85 | c-Pr | 1 | CH₂c-Pr | c-Bu |
| 3-86 | c-Pr | 2 | CH₂c-Pr | c-Bu |
| 3-87 | c-Pr | 0 | CH₂CH₂OMe | c-Bu |
| 3-88 | c-Pr | 1 | CH₂CH₂OMe | c-Bu |
| 3-89 | c-Pr | 2 | CH₂CH₂OMe | c-Bu |

**Tabelle 4: Erfindungsgemäße Verbindungen der allgemeinen Formel (I), worin Q für Q2 und R^{x} für eine Methylgruppe stehen**

| | | | | |
|---|---|---|---|---|
| 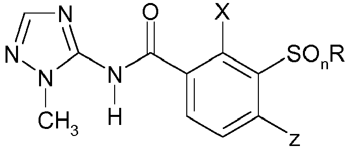 | | | | |

| Nr. | X | n | R | Z |
|---|---|---|---|---|
| 4-1 | Me | 0 | Me | c-Pr |
| 4-2 | Me | 1 | Me | c-Pr |
| 4-3 | Me | 2 | Me | c-Pr |
| 4-4 | Me | 0 | Et | c-Pr |
| 4-5 | Me | 1 | Et | c-Pr |
| 4-6 | Me | 2 | Et | c-Pr |
| 4-7 | Me | 0 | c-Pr | c-Pr |
| 4-8 | Me | 1 | c-Pr | c-Pr |
| 4-9 | Me | 2 | c-Pr | c-Pr |
| 4-10 | Me | 0 | CH₂c-Pr | c-Pr |
| 4-11 | Me | 1 | CH₂c-Pr | c-Pr |
| 4-12 | Me | 2 | CH₂c-Pr | c-Pr |
| 4-13 | Me | 0 | CH₂CH₂OMe | c-Pr |
| 4-14 | Me | 1 | CH₂CH₂OMe | c-Pr |
| 4-15 | Me | 2 | CH₂CH₂OMe | c-Pr |
| 4-16 | Me | 0 | Me | c-Bu |
| 4-17 | Me | 1 | Me | c-Bu |
| 4-18 | Me | 2 | Me | c-Bu |
| 4-19 | Me | 0 | Et | c-Bu |
| 4-20 | Me | 1 | Et | c-Bu |
| 4-21 | Me | 2 | Et | c-Bu |
| 4-22 | Me | 0 | c-Pr | c-Bu |
| 4-23 | Me | 1 | c-Pr | c-Bu |
| 4-24 | Me | 2 | c-Pr | c-Bu |
| 4-25 | Me | 0 | CH₂c-Pr | c-Bu |
| 4-26 | Me | 1 | CH₂c-Pr | c-Bu |
| 4-27 | Me | 2 | CH₂c-Pr | c-Bu |
| 4-28 | Me | 0 | CH₂CH₂OMe | c-Bu |
| 4-29 | Me | 1 | CH₂CH₂OMe | c-Bu |
| 4-30 | Me | 2 | CH₂CH₂OMe | c-Bu |
| 4-31 | Et | 0 | Me | c-Pr |
| 4-32 | Et | 1 | Me | c-Pr |
| 4-33 | Et | 2 | Me | c-Pr |
| 4-34 | Et | 0 | Et | c-Pr |
| 4-35 | Et | 1 | Et | c-Pr |
| 4-36 | Et | 2 | Et | c-Pr |
| 4-37 | Et | 0 | c-Pr | c-Pr |
| 4-38 | Et | 1 | c-Pr | c-Pr |
| 4-39 | Et | 2 | c-Pr | c-Pr |
| 4-40 | Et | 0 | CH₂c-Pr | c-Pr |
| 4-41 | Et | 1 | CH₂c-Pr | c-Pr |
| 4-42 | Et | 2 | CH₂c-Pr | c-Pr |
| 4-43 | Et | 0 | CH₂CH₂OMe | c-Pr |
| 4-44 | Et | 1 | CH₂CH₂OMe | c-Pr |
| 4-45 | Et | 2 | CH₂CH₂OMe | c-Pr |
| 4-46 | Et | 0 | Me | c-Bu |
| 4-47 | Et | 1 | Me | c-Bu |
| 4-48 | Et | 2 | Me | c-Bu |
| 4-49 | Et | 0 | Et | c-Bu |
| 4-50 | Et | 1 | Et | c-Bu |
| 4-51 | Et | 2 | Et | c-Bu |
| 4-52 | Et | 0 | c-Pr | c-Bu |
| 4-53 | Et | 1 | c-Pr | c-Bu |
| 4-54 | Et | 2 | c-Pr | c-Bu |
| 4-55 | Et | 0 | CH₂c-Pr | c-Bu |
| 4-56 | Et | 1 | CH₂c-Pr | c-Bu |
| 4-57 | Et | 2 | CH₂c-Pr | c-Bu |
| 4-58 | Et | 0 | CH₂CH₂OMe | c-Bu |
| 4-59 | Et | 1 | CH₂CH₂OMe | c-Bu |
| 4-60 | Et | 2 | CH₂CH₂OMe | c-Bu |
| 4-61 | c-Pr | 0 | Me | c-Pr |
| 4-62 | c-Pr | 1 | Me | c-Pr |
| 4-63 | c-Pr | 2 | Me | c-Pr |
| 4-64 | c-Pr | 0 | Et | c-Pr |
| 4-65 | c-Pr | 1 | Et | c-Pr |
| 4-66 | c-Pr | 2 | Et | c-Pr |
| 4-67 | c-Pr | 0 | c-Pr | c-Pr |
| 4-68 | c-Pr | 1 | c-Pr | c-Pr |
| 4-69 | c-Pr | 2 | c-Pr | c-Pr |
| 4-70 | c-Pr | 0 | CH₂c-Pr | c-Pr |
| 4-71 | c-Pr | 1 | CH₂c-Pr | c-Pr |
| 4-72 | c-Pr | 2 | CH₂c-Pr | c-Pr |
| 4-73 | c-Pr | 0 | CH₂CH₂OMe | c-Pr |
| 4-74 | c-Pr | 1 | CH₂CH₂OMe | c-Pr |
| 4-75 | c-Pr | 2 | CH₂CH₂OMe | c-Pr |
| 4-76 | c-Pr | 0 | Me | c-Bu |
| 4-77 | c-Pr | 1 | Me | c-Bu |
| 4-78 | c-Pr | 2 | Me | c-Bu |
| 4-79 | c-Pr | 0 | Et | c-Bu |
| 4-80 | c-Pr | 1 | Et | c-Bu |
| 4-81 | c-Pr | 2 | Et | c-Bu |
| 4-82 | c-Pr | 0 | c-Pr | c-Bu |
| 4-83 | c-Pr | 1 | c-Pr | c-Bu |
| 4-84 | c-Pr | 2 | c-Pr | c-Bu |
| 4-85 | c-Pr | 0 | CH₂c-Pr | c-Bu |
| 4-86 | c-Pr | 1 | CH₂c-Pr | c-Bu |
| 4-87 | c-Pr | 2 | CH₂c-Pr | c-Bu |
| 4-88 | c-Pr | 0 | CH₂CH₂OMe | c-Bu |
| 4-89 | c-Pr | 1 | CH₂CH₂OMe | c-Bu |
| 4-90 | c-Pr | 2 | CH₂CH₂OMe | c-Bu |

**Tabelle 5: Erfindungsgemäße Verbindungen der allgemeinen Formel (I), worin Q für Q3 und R^{y} für eine Methylgruppe stehen**

| | | | | |
|---|---|---|---|---|
| 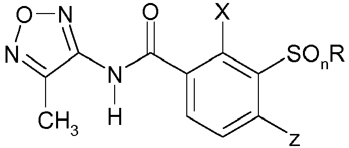 | | | | |

| Nr. | X | n | R | Z |
|---|---|---|---|---|
| 5-1 | Me | 0 | Me | c-Pr |
| 5-2 | Me | 1 | Me | c-Pr |
| 5-3 | Me | 2 | Me | c-Pr |
| 5-4 | Me | 0 | Et | c-Pr |
| 5-5 | Me | 1 | Et | c-Pr |
| 5-6 | Me | 2 | Et | c-Pr |
| 5-7 | Me | 0 | c-Pr | c-Pr |
| 5-8 | Me | 1 | c-Pr | c-Pr |
| 5-9 | Me | 2 | c-Pr | c-Pr |
| 5-10 | Me | 0 | CH₂c-Pr | c-Pr |
| 5-11 | Me | 1 | CH₂c-Pr | c-Pr |
| 5-12 | Me | 2 | CH₂c-Pr | c-Pr |
| 5-13 | Me | 0 | CH₂CH₂OMe | c-Pr |
| 5-14 | Me | 1 | CH₂CH₂OMe | c-Pr |
| 5-15 | Me | 2 | CH₂CH₂OMe | c-Pr |
| 5-16 | Me | 0 | Me | c-Bu |
| 5-17 | Me | 1 | Me | c-Bu |
| 5-18 | Me | 2 | Me | c-Bu |
| 5-19 | Me | 0 | Et | c-Bu |
| 5-20 | Me | 1 | Et | c-Bu |
| 5-21 | Me | 2 | Et | c-Bu |
| 5-22 | Me | 0 | c-Pr | c-Bu |
| 5-23 | Me | 1 | c-Pr | c-Bu |
| 5-24 | Me | 2 | c-Pr | c-Bu |
| 5-25 | Me | 0 | CH₂c-Pr | c-Bu |
| 5-26 | Me | 1 | CH₂c-Pr | c-Bu |
| 5-27 | Me | 2 | CH₂c-Pr | c-Bu |
| 5-28 | Me | 0 | CH₂CH₂OMe | c-Bu |
| 5-29 | Me | 1 | CH₂CH₂OMe | c-Bu |
| 5-30 | Me | 2 | CH₂CH₂OMe | c-Bu |
| 5-31 | Et | 0 | Me | c-Pr |
| 5-32 | Et | 1 | Me | c-Pr |
| 5-33 | Et | 2 | Me | c-Pr |
| 5-34 | Et | 0 | Et | c-Pr |
| 5-35 | Et | 1 | Et | c-Pr |
| 5-36 | Et | 2 | Et | c-Pr |
| 5-37 | Et | 0 | c-Pr | c-Pr |
| 5-38 | Et | 1 | c-Pr | c-Pr |
| 5-39 | Et | 2 | c-Pr | c-Pr |
| 5-40 | Et | 0 | CH₂c-Pr | c-Pr |
| 5-41 | Et | 1 | CH₂c-Pr | c-Pr |
| 5-42 | Et | 2 | CH₂c-Pr | c-Pr |
| 5-43 | Et | 0 | CH₂CH₂OMe | c-Pr |
| 5-44 | Et | 1 | CH₂CH₂OMe | c-Pr |
| 5-45 | Et | 2 | CH₂CH₂OMe | c-Pr |
| 5-46 | Et | 0 | Me | c-Bu |
| 5-47 | Et | 1 | Me | c-Bu |
| 5-48 | Et | 2 | Me | c-Bu |
| 5-49 | Et | 0 | Et | c-Bu |
| 5-50 | Et | 1 | Et | c-Bu |
| 5-51 | Et | 2 | Et | c-Bu |
| 5-52 | Et | 0 | c-Pr | c-Bu |
| 5-53 | Et | 1 | c-Pr | c-Bu |
| 5-54 | Et | 2 | c-Pr | c-Bu |
| 5-55 | Et | 0 | CH₂c-Pr | c-Bu |
| 5-56 | Et | 1 | CH₂c-Pr | c-Bu |
| 5-57 | Et | 2 | CH₂c-Pr | c-Bu |
| 5-58 | Et | 0 | CH₂CH₂OMe | c-Bu |
| 5-59 | Et | 1 | CH₂CH₂OMe | c-Bu |
| 5-60 | Et | 2 | CH₂CH₂OMe | c-Bu |
| 5-61 | c-Pr | 0 | Me | c-Pr |
| 5-62 | c-Pr | 1 | Me | c-Pr |
| 5-63 | c-Pr | 2 | Me | c-Pr |
| 5-64 | c-Pr | 0 | Et | c-Pr |
| 5-65 | c-Pr | 1 | Et | c-Pr |
| 5-66 | c-Pr | 2 | Et | c-Pr |
| 5-67 | c-Pr | 0 | c-Pr | c-Pr |
| 5-68 | c-Pr | 1 | c-Pr | c-Pr |
| 5-69 | c-Pr | 2 | c-Pr | c-Pr |
| 5-70 | c-Pr | 0 | CH₂c-Pr | c-Pr |
| 5-71 | c-Pr | 1 | CH₂c-Pr | c-Pr |
| 5-72 | c-Pr | 2 | CH₂c-Pr | c-Pr |
| 5-73 | c-Pr | 0 | CH₂CH₂OMe | c-Pr |
| 5-74 | c-Pr | 1 | CH₂CH₂OMe | c-Pr |
| 5-75 | c-Pr | 2 | CH₂CH₂OMe | c-Pr |
| 5-76 | c-Pr | 0 | Me | c-Bu |
| 5-77 | c-Pr | 1 | Me | c-Bu |
| 5-78 | c-Pr | 2 | Me | c-Bu |
| 5-79 | c-Pr | 0 | Et | c-Bu |
| 5-80 | c-Pr | 1 | Et | c-Bu |
| 5-81 | c-Pr | 2 | Et | c-Bu |
| 5-82 | c-Pr | 0 | c-Pr | c-Bu |
| 5-83 | c-Pr | 1 | c-Pr | c-Bu |
| 5-84 | c-Pr | 2 | c-Pr | c-Bu |
| 5-85 | c-Pr | 0 | CH₂c-Pr | c-Bu |
| 5-86 | c-Pr | 1 | CH₂c-Pr | c-Bu |
| 5-87 | c-Pr | 2 | CH₂c-Pr | c-Bu |
| 5-88 | c-Pr | 0 | CH₂CH₂OMe | c-Bu |
| 5-89 | c-Pr | 1 | CH₂CH₂OMe | c-Bu |
| 5-90 | c-Pr | 2 | CH₂CH₂OMe | c-Bu |

**Tabelle 6: Erfindungsgemäße Verbindungen der allgemeinen Formel (I), worin Q für Q3 und R^{y} für ein Chloratom stehen**

| | | | | |
|---|---|---|---|---|
| 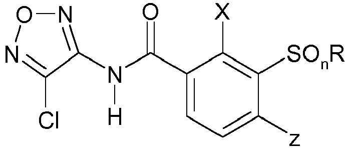 | | | | |

| Nr. | X | n | R | Z |
|---|---|---|---|---|
| 6-1 | Me | 0 | Me | c-Pr |
| 6-2 | Me | 1 | Me | c-Pr |
| 6-3 | Me | 2 | Me | c-Pr |
| 6-4 | Me | 0 | Et | c-Pr |
| 6-5 | Me | 1 | Et | c-Pr |
| 6-6 | Me | 2 | Et | c-Pr |
| 6-7 | Me | 0 | c-Pr | c-Pr |
| 6-8 | Me | 1 | c-Pr | c-Pr |
| 6-9 | Me | 2 | c-Pr | c-Pr |
| 6-10 | Me | 0 | CH₂c-Pr | c-Pr |
| 6-11 | Me | 1 | CH₂c-Pr | c-Pr |
| 6-12 | Me | 2 | CH₂c-Pr | c-Pr |
| 6-13 | Me | 0 | CH₂CH₂OMe | c-Pr |
| 6-14 | Me | 1 | CH₂CH₂OMe | c-Pr |
| 6-15 | Me | 2 | CH₂CH₂OMe | c-Pr |
| 6-16 | Me | 0 | Me | c-Bu |
| 6-17 | Me | 1 | Me | c-Bu |
| 6-18 | Me | 2 | Me | c-Bu |
| 6-19 | Me | 0 | Et | c-Bu |
| 6-20 | Me | 1 | Et | c-Bu |
| 6-21 | Me | 2 | Et | c-Bu |
| 6-22 | Me | 0 | c-Pr | c-Bu |
| 6-23 | Me | 1 | c-Pr | c-Bu |
| 6-24 | Me | 2 | c-Pr | c-Bu |
| 6-25 | Me | 0 | CH₂c-Pr | c-Bu |
| 6-26 | Me | 1 | CH₂c-Pr | c-Bu |
| 6-27 | Me | 2 | CH₂c-Pr | c-Bu |
| 6-28 | Me | 0 | CH₂CH₂OMe | c-Bu |
| 6-29 | Me | 1 | CH₂CH₂OMe | c-Bu |
| 6-30 | Me | 2 | CH₂CH₂OMe | c-Bu |
| 6-31 | Et | 0 | Me | c-Pr |
| 6-32 | Et | 1 | Me | c-Pr |
| 6-33 | Et | 2 | Me | c-Pr |
| 6-34 | Et | 0 | Et | c-Pr |
| 6-35 | Et | 1 | Et | c-Pr |
| 6-36 | Et | 2 | Et | c-Pr |
| 6-37 | Et | 0 | c-Pr | c-Pr |
| 6-38 | Et | 1 | c-Pr | c-Pr |
| 6-39 | Et | 2 | c-Pr | c-Pr |
| 6-40 | Et | 0 | CH₂c-Pr | c-Pr |
| 6-41 | Et | 1 | CH₂c-Pr | c-Pr |
| 6-42 | Et | 2 | CH₂c-Pr | c-Pr |
| 6-43 | Et | 0 | CH₂CH₂OMe | c-Pr |
| 6-44 | Et | 1 | CH₂CH₂OMe | c-Pr |
| 6-45 | Et | 2 | CH₂CH₂OMe | c-Pr |
| 6-46 | Et | 0 | Me | c-Bu |
| 6-47 | Et | 1 | Me | c-Bu |
| 6-48 | Et | 2 | Me | c-Bu |
| 6-49 | Et | 0 | Et | c-Bu |
| 6-50 | Et | 1 | Et | c-Bu |
| 6-51 | Et | 2 | Et | c-Bu |
| 6-52 | Et | 0 | c-Pr | c-Bu |
| 6-53 | Et | 1 | c-Pr | c-Bu |
| 6-54 | Et | 2 | c-Pr | c-Bu |
| 6-55 | Et | 0 | CH₂c-Pr | c-Bu |
| 6-56 | Et | 1 | CH₂c-Pr | c-Bu |
| 6-57 | Et | 2 | CH₂c-Pr | c-Bu |
| 6-58 | Et | 0 | CH₂CH₂OMe | c-Bu |
| 6-59 | Et | 1 | CH₂CH₂OMe | c-Bu |
| 6-60 | Et | 2 | CH₂CH₂OMe | c-Bu |
| 6-61 | c-Pr | 0 | Me | c-Pr |
| 6-62 | c-Pr | 1 | Me | c-Pr |
| 6-63 | c-Pr | 2 | Me | c-Pr |
| 6-64 | c-Pr | 0 | Et | c-Pr |
| 6-65 | c-Pr | 1 | Et | c-Pr |
| 6-66 | c-Pr | 2 | Et | c-Pr |
| 6-67 | c-Pr | 0 | c-Pr | c-Pr |
| 6-68 | c-Pr | 1 | c-Pr | c-Pr |
| 6-69 | c-Pr | 2 | c-Pr | c-Pr |
| 6-70 | c-Pr | 0 | CH₂c-Pr | c-Pr |
| 6-71 | c-Pr | 1 | CH₂c-Pr | c-Pr |
| 6-72 | c-Pr | 2 | CH₂c-Pr | c-Pr |
| 6-73 | c-Pr | 0 | CH₂CH₂OMe | c-Pr |
| 6-74 | c-Pr | 1 | CH₂CH₂OMe | c-Pr |
| 6-75 | c-Pr | 2 | CH₂CH₂OMe | c-Pr |
| 6-76 | c-Pr | 0 | Me | c-Bu |
| 6-77 | c-Pr | 1 | Me | c-Bu |
| 6-78 | c-Pr | 2 | Me | c-Bu |
| 6-79 | c-Pr | 0 | Et | c-Bu |
| 6-80 | c-Pr | 1 | Et | c-Bu |
| 6-81 | c-Pr | 2 | Et | c-Bu |
| 6-82 | c-Pr | 0 | c-Pr | c-Bu |
| 6-83 | c-Pr | 1 | c-Pr | c-Bu |
| 6-84 | c-Pr | 2 | c-Pr | c-Bu |
| 6-85 | c-Pr | 0 | CH₂c-Pr | c-Bu |
| 6-86 | c-Pr | 1 | CH₂c-Pr | c-Bu |
| 6-87 | c-Pr | 2 | CH₂c-Pr | c-Bu |
| 6-88 | c-Pr | 0 | CH₂CH₂OMe | c-Bu |
| 6-89 | c-Pr | 1 | CH₂CH₂OMe | c-Bu |
| 6-90 | c-Pr | 2 | CH₂CH₂OMe | c-Bu |

**Tabelle 7: Erfindungsgemäße Verbindungen der allgemeinen Formel (I), worin Q für Q4 und R^{z} für eine Methylgruppe stehen**

| | | | | |
|---|---|---|---|---|
| 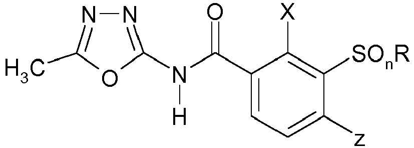 | | | | |

| Nr. | X | n | R | Z |
|---|---|---|---|---|
| 7-1 | Me | 0 | Me | c-Pr |
| 7-2 | Me | 1 | Me | c-Pr |
| 7-3 | Me | 2 | Me | c-Pr |
| 7-4 | Me | 0 | Et | c-Pr |
| 7-5 | Me | 1 | Et | c-Pr |
| 7-6 | Me | 2 | Et | c-Pr |
| 7-7 | Me | 0 | c-Pr | c-Pr |
| 7-8 | Me | 1 | c-Pr | c-Pr |
| 7-9 | Me | 2 | c-Pr | c-Pr |
| 7-10 | Me | 0 | CH₂c-Pr | c-Pr |
| 7-11 | Me | 1 | CH₂c-Pr | c-Pr |
| 7-12 | Me | 2 | CH₂c-Pr | c-Pr |
| 7-13 | Me | 0 | CH₂CH₂OMe | c-Pr |
| 7-14 | Me | 1 | CH₂CH₂OMe | c-Pr |
| 7-15 | Me | 2 | CH₂CH₂OMe | c-Pr |
| 7-16 | Me | 0 | Me | c-Bu |
| 7-17 | Me | 1 | Me | c-Bu |
| 7-18 | Me | 2 | Me | c-Bu |
| 7-19 | Me | 0 | Et | c-Bu |
| 7-20 | Me | 1 | Et | c-Bu |
| 7-21 | Me | 2 | Et | c-Bu |
| 7-22 | Me | 0 | c-Pr | c-Bu |
| 7-23 | Me | 1 | c-Pr | c-Bu |
| 7-24 | Me | 2 | c-Pr | c-Bu |
| 7-25 | Me | 0 | CH₂c-Pr | c-Bu |
| 7-26 | Me | 1 | CH₂c-Pr | c-Bu |
| 7-27 | Me | 2 | CH₂c-Pr | c-Bu |
| 7-28 | Me | 0 | CH₂CH₂OMe | c-Bu |
| 7-29 | Me | 1 | CH₂CH₂OMe | c-Bu |
| 7-30 | Me | 2 | CH₂CH₂OMe | c-Bu |
| 7-31 | Et | 0 | Me | c-Pr |
| 7-32 | Et | 1 | Me | c-Pr |
| 7-33 | Et | 2 | Me | c-Pr |
| 7-34 | Et | 0 | Et | c-Pr |
| 7-35 | Et | 1 | Et | c-Pr |
| 7-36 | Et | 2 | Et | c-Pr |
| 7-37 | Et | 0 | c-Pr | c-Pr |
| 7-38 | Et | 1 | c-Pr | c-Pr |
| 7-39 | Et | 2 | c-Pr | c-Pr |
| 7-40 | Et | 0 | CH₂c-Pr | c-Pr |
| 7-41 | Et | 1 | CH₂c-Pr | c-Pr |
| 7-42 | Et | 2 | CH₂c-Pr | c-Pr |
| 7-43 | Et | 0 | CH₂CH₂OMe | c-Pr |
| 7-44 | Et | 1 | CH₂CH₂OMe | c-Pr |
| 7-45 | Et | 2 | CH₂CH₂OMe | c-Pr |
| 7-46 | Et | 0 | Me | c-Bu |
| 7-47 | Et | 1 | Me | c-Bu |
| 7-48 | Et | 2 | Me | c-Bu |
| 7-49 | Et | 0 | Et | c-Bu |
| 7-50 | Et | 1 | Et | c-Bu |
| 7-51 | Et | 2 | Et | c-Bu |
| 7-52 | Et | 0 | c-Pr | c-Bu |
| 7-53 | Et | 1 | c-Pr | c-Bu |
| 7-54 | Et | 2 | c-Pr | c-Bu |
| 7-55 | Et | 0 | CH₂c-Pr | c-Bu |
| 7-56 | Et | 1 | CH₂c-Pr | c-Bu |
| 7-57 | Et | 2 | CH₂c-Pr | c-Bu |
| 7-58 | Et | 0 | CH₂CH₂OMe | c-Bu |
| 7-59 | Et | 1 | CH₂CH₂OMe | c-Bu |
| 7-60 | Et | 2 | CH₂CH₂OMe | c-Bu |
| 7-61 | c-Pr | 0 | Me | c-Pr |
| 7-62 | c-Pr | 1 | Me | c-Pr |
| 7-63 | c-Pr | 2 | Me | c-Pr |
| 7-64 | c-Pr | 0 | Et | c-Pr |
| 7-65 | c-Pr | 1 | Et | c-Pr |
| 7-66 | c-Pr | 2 | Et | c-Pr |
| 7-67 | c-Pr | 0 | c-Pr | c-Pr |
| 7-68 | c-Pr | 1 | c-Pr | c-Pr |
| 7-69 | c-Pr | 2 | c-Pr | c-Pr |
| 7-70 | c-Pr | 0 | CH₂c-Pr | c-Pr |
| 7-71 | c-Pr | 1 | CH₂c-Pr | c-Pr |
| 7-72 | c-Pr | 2 | CH₂c-Pr | c-Pr |
| 7-73 | c-Pr | 0 | CH₂CH₂OMe | c-Pr |
| 7-74 | c-Pr | 1 | CH₂CH₂OMe | c-Pr |
| 7-75 | c-Pr | 2 | CH₂CH₂OMe | c-Pr |
| 7-76 | c-Pr | 0 | Me | c-Bu |
| 7-77 | c-Pr | 1 | Me | c-Bu |
| 7-78 | c-Pr | 2 | Me | c-Bu |
| 7-79 | c-Pr | 0 | Et | c-Bu |
| 7-80 | c-Pr | 1 | Et | c-Bu |
| 7-81 | c-Pr | 2 | Et | c-Bu |
| 7-82 | c-Pr | 0 | c-Pr | c-Bu |
| 7-83 | c-Pr | 1 | c-Pr | c-Bu |
| 7-84 | c-Pr | 2 | c-Pr | c-Bu |
| 7-85 | c-Pr | 0 | CH₂c-Pr | c-Bu |
| 7-86 | c-Pr | 1 | CH₂c-Pr | c-Bu |
| 7-87 | c-Pr | 2 | CH₂c-Pr | c-Bu |
| 7-88 | c-Pr | 0 | CH₂CH₂OMe | c-Bu |
| 7-89 | c-Pr | 1 | CH₂CH₂OMe | c-Bu |
| 7-90 | c-Pr | 2 | CH₂CH₂OMe | c-Bu |

**Tabelle 8: Erfindungsgemäße Verbindungen der allgemeinen Formel (I) in Form der Natriumsalze, worin Q für Q1 und R^{x} für eine Methylgruppe stehen**

| | | | | |
|---|---|---|---|---|
| 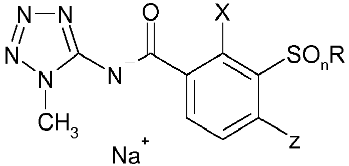 | | | | |

| Nr. | X | n | R | Z |
|---|---|---|---|---|
| 8-1 | Me | 0 | Me | c-Pr |
| 8-2 | Me | 1 | Me | c-Pr |
| 8-3 | Me | 2 | Me | c-Pr |
| 8-4 | Me | 0 | Et | c-Pr |
| 8-5 | Me | 1 | Et | c-Pr |
| 8-6 | Me | 2 | Et | c-Pr |
| 8-7 | Me | 0 | c-Pr | c-Pr |
| 8-8 | Me | 1 | c-Pr | c-Pr |
| 8-9 | Me | 2 | c-Pr | c-Pr |
| 8-10 | Me | 0 | CH₂c-Pr | c-Pr |
| 8-11 | Me | 1 | CH₂c-Pr | c-Pr |
| 8-12 | Me | 2 | CH₂c-Pr | c-Pr |
| 8-13 | Me | 0 | CH₂CH₂OMe | c-Pr |
| 8-14 | Me | 1 | CH₂CH₂OMe | c-Pr |
| 8-15 | Me | 2 | CH₂CH₂OMe | c-Pr |
| 8-16 | Me | 0 | Me | c-Bu |
| 8-17 | Me | 1 | Me | c-Bu |
| 8-18 | Me | 2 | Me | c-Bu |
| 8-19 | Me | 0 | Et | c-Bu |
| 8-20 | Me | 1 | Et | c-Bu |
| 8-21 | Me | 2 | Et | c-Bu |
| 8-22 | Me | 0 | c-Pr | c-Bu |
| 8-23 | Me | 1 | c-Pr | c-Bu |
| 8-24 | Me | 2 | c-Pr | c-Bu |
| 8-25 | Me | 0 | CH₂c-Pr | c-Bu |
| 8-26 | Me | 1 | CH₂c-Pr | c-Bu |
| 8-27 | Me | 2 | CH₂c-Pr | c-Bu |
| 8-28 | Me | 0 | CH₂CH₂OMe | c-Bu |
| 8-29 | Me | 1 | CH₂CH₂OMe | c-Bu |
| 8-30 | Me | 2 | CH₂CH₂OMe | c-Bu |
| 8-31 | Et | 0 | Me | c-Pr |
| 8-32 | Et | 1 | Me | c-Pr |
| 8-33 | Et | 2 | Me | c-Pr |
| 8-34 | Et | 0 | Et | c-Pr |
| 8-35 | Et | 1 | Et | c-Pr |
| 8-36 | Et | 2 | Et | c-Pr |
| 8-37 | Et | 0 | c-Pr | c-Pr |
| 8-38 | Et | 1 | c-Pr | c-Pr |
| 8-39 | Et | 2 | c-Pr | c-Pr |
| 8-40 | Et | 0 | CH₂c-Pr | c-Pr |
| 8-41 | Et | 1 | CH₂c-Pr | c-Pr |
| 8-42 | Et | 2 | CH₂c-Pr | c-Pr |
| 8-43 | Et | 0 | CH₂CH₂OMe | c-Pr |
| 8-44 | Et | 1 | CH₂CH₂OMe | c-Pr |
| 8-45 | Et | 2 | CH₂CH₂OMe | c-Pr |
| 8-46 | Et | 0 | Me | c-Bu |
| 8-47 | Et | 1 | Me | c-Bu |
| 8-48 | Et | 2 | Me | c-Bu |
| 8-49 | Et | 0 | Et | c-Bu |
| 8-50 | Et | 1 | Et | c-Bu |
| 8-51 | Et | 2 | Et | c-Bu |
| 8-52 | Et | 0 | c-Pr | c-Bu |
| 8-53 | Et | 1 | c-Pr | c-Bu |
| 8-54 | Et | 2 | c-Pr | c-Bu |
| 8-55 | Et | 0 | CH₂c-Pr | c-Bu |
| 8-56 | Et | 1 | CH₂c-Pr | c-Bu |
| 8-57 | Et | 2 | CH₂c-Pr | c-Bu |
| 8-58 | Et | 0 | CH₂CH₂OMe | c-Bu |
| 8-59 | Et | 1 | CH₂CH₂OMe | c-Bu |
| 8-60 | Et | 2 | CH₂CH₂OMe | c-Bu |
| 8-61 | c-Pr | 0 | Me | c-Pr |
| 8-62 | c-Pr | 1 | Me | c-Pr |
| 8-63 | c-Pr | 2 | Me | c-Pr |
| 8-64 | c-Pr | 0 | Et | c-Pr |
| 8-65 | c-Pr | 1 | Et | c-Pr |
| 8-66 | c-Pr | 2 | Et | c-Pr |
| 8-67 | c-Pr | 0 | c-Pr | c-Pr |
| 8-68 | c-Pr | 1 | c-Pr | c-Pr |
| 8-69 | c-Pr | 2 | c-Pr | c-Pr |
| 8-70 | c-Pr | 0 | CH₂c-Pr | c-Pr |
| 8-71 | c-Pr | 1 | CH₂c-Pr | c-Pr |
| 8-72 | c-Pr | 2 | CH₂c-Pr | c-Pr |
| 8-73 | c-Pr | 0 | CH₂CH₂OMe | c-Pr |
| 8-74 | c-Pr | 1 | CH₂CH₂OMe | c-Pr |
| 8-75 | c-Pr | 2 | CH₂CH₂OMe | c-Pr |
| 8-76 | c-Pr | 0 | Me | c-Bu |
| 8-77 | c-Pr | 1 | Me | c-Bu |
| 8-78 | c-Pr | 2 | Me | c-Bu |
| 8-79 | c-Pr | 0 | Et | c-Bu |
| 8-80 | c-Pr | 1 | Et | c-Bu |
| 8-81 | c-Pr | 2 | Et | c-Bu |
| 8-82 | c-Pr | 0 | c-Pr | c-Bu |
| 8-83 | c-Pr | 1 | c-Pr | c-Bu |
| 8-84 | c-Pr | 2 | c-Pr | c-Bu |
| 8-85 | c-Pr | 0 | CH₂c-Pr | c-Bu |
| 8-86 | c-Pr | 1 | CH₂c-Pr | c-Bu |
| 8-87 | c-Pr | 2 | CH₂c-Pr | c-Bu |
| 8-88 | c-Pr | 0 | CH₂CH₂OMe | c-Bu |
| 8-89 | c-Pr | 1 | CH₂CH₂OMe | c-Bu |
| 8-90 | c-Pr | 2 | CH₂CH₂OMe | c-Bu |

**Tabelle 9: Erfindungsgemäße Benzoesäuren der Formel (II)**

| | | | | |
|---|---|---|---|---|
| 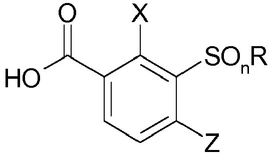 | | | | |

| Nr. | X | n | R | Z |
|---|---|---|---|---|
| 9-1 | Me | 0 | Me | c-Pr |
| 9-2 | Me | 1 | Me | c-Pr |
| 9-3 | Me | 2 | Me | c-Pr |
| 9-4 | Me | 0 | Et | c-Pr |
| 9-5 | Me | 1 | Et | c-Pr |
| 9-6 | Me | 2 | Et | c-Pr |
| 9-7 | Me | 0 | c-Pr | c-Pr |
| 9-8 | Me | 1 | c-Pr | c-Pr |
| 9-9 | Me | 2 | c-Pr | c-Pr |
| 9-10 | Me | 0 | CH₂c-Pr | c-Pr |
| 9-11 | Me | 1 | CH₂c-Pr | c-Pr |
| 9-12 | Me | 2 | CH₂c-Pr | c-Pr |
| 9-13 | Me | 0 | CH₂CH₂OMe | c-Pr |
| 9-14 | Me | 1 | CH₂CH₂OMe | c-Pr |
| 9-15 | Me | 2 | CH₂CH₂OMe | c-Pr |
| 9-16 | Me | 0 | Me | c-Bu |
| 9-17 | Me | 1 | Me | c-Bu |
| 9-18 | Me | 2 | Me | c-Bu |
| 9-19 | Me | 0 | Et | c-Bu |
| 9-20 | Me | 1 | Et | c-Bu |
| 9-21 | Me | 2 | Et | c-Bu |
| 9-22 | Me | 0 | c-Pr | c-Bu |
| 9-23 | Me | 1 | c-Pr | c-Bu |
| 9-24 | Me | 2 | c-Pr | c-Bu |
| 9-25 | Me | 0 | CH₂c-Pr | c-Bu |
| 9-26 | Me | 1 | CH₂c-Pr | c-Bu |
| 9-27 | Me | 2 | CH₂c-Pr | c-Bu |
| 9-28 | Me | 0 | CH₂CH₂OMe | c-Bu |
| 9-29 | Me | 1 | CH₂CH₂OMe | c-Bu |
| 9-30 | Me | 2 | CH₂CH₂OMe | c-Bu |
| 9-31 | Et | 0 | Me | c-Pr |
| 9-32 | Et | 1 | Me | c-Pr |
| 9-33 | Et | 2 | Me | c-Pr |
| 9-34 | Et | 0 | Et | c-Pr |
| 9-35 | Et | 1 | Et | c-Pr |
| 9-36 | Et | 2 | Et | c-Pr |
| 9-37 | Et | 0 | c-Pr | c-Pr |
| 9-38 | Et | 1 | c-Pr | c-Pr |
| 9-39 | Et | 2 | c-Pr | c-Pr |
| 9-40 | Et | 0 | CH₂c-Pr | c-Pr |
| 9-41 | Et | 1 | CH₂c-Pr | c-Pr |
| 9-42 | Et | 2 | CH₂c-Pr | c-Pr |
| 9-43 | Et | 0 | CH₂CH₂OMe | c-Pr |
| 9-44 | Et | 1 | CH₂CH₂OMe | c-Pr |
| 9-45 | Et | 2 | CH₂CH₂OMe | c-Pr |
| 9-46 | Et | 0 | Me | c-Bu |
| 9-47 | Et | 1 | Me | c-Bu |
| 9-48 | Et | 2 | Me | c-Bu |
| 9-49 | Et | 0 | Et | c-Bu |
| 9-50 | Et | 1 | Et | c-Bu |
| 9-51 | Et | 2 | Et | c-Bu |
| 9-52 | Et | 0 | c-Pr | c-Bu |
| 9-53 | Et | 1 | c-Pr | c-Bu |
| 9-54 | Et | 2 | c-Pr | c-Bu |
| 9-55 | Et | 0 | CH₂c-Pr | c-Bu |
| 9-56 | Et | 1 | CH₂c-Pr | c-Bu |
| 9-57 | Et | 2 | CH₂c-Pr | c-Bu |
| 9-58 | Et | 0 | CH₂CH₂OMe | c-Bu |
| 9-59 | Et | 1 | CH₂CH₂OMe | c-Bu |
| 9-60 | Et | 2 | CH₂CH₂OMe | c-Bu |
| 9-61 | c-Pr | 0 | Me | c-Pr |
| 9-62 | c-Pr | 1 | Me | c-Pr |
| 9-63 | c-Pr | 2 | Me | c-Pr |
| 9-64 | c-Pr | 0 | Et | c-Pr |
| 9-65 | c-Pr | 1 | Et | c-Pr |
| 9-66 | c-Pr | 2 | Et | c-Pr |
| 9-67 | c-Pr | 0 | c-Pr | c-Pr |
| 9-68 | c-Pr | 1 | c-Pr | c-Pr |
| 9-69 | c-Pr | 2 | c-Pr | c-Pr |
| 9-70 | c-Pr | 0 | CH₂c-Pr | c-Pr |
| 9-71 | c-Pr | 1 | CH₂c-Pr | c-Pr |
| 9-72 | c-Pr | 2 | CH₂c-Pr | c-Pr |
| 9-73 | c-Pr | 0 | CH₂CH₂OMe | c-Pr |
| 9-74 | c-Pr | 1 | CH₂CH₂OMe | c-Pr |
| 9-75 | c-Pr | 2 | CH₂CH₂OMe | c-Pr |
| 9-76 | c-Pr | 0 | Me | c-Bu |
| 9-77 | c-Pr | 1 | Me | c-Bu |
| 9-78 | c-Pr | 2 | Me | c-Bu |
| 9-79 | c-Pr | 0 | Et | c-Bu |
| 9-80 | c-Pr | 1 | Et | c-Bu |
| 9-81 | c-Pr | 2 | Et | c-Bu |
| 9-82 | c-Pr | 0 | c-Pr | c-Bu |
| 9-83 | c-Pr | 1 | c-Pr | c-Bu |
| 9-84 | c-Pr | 2 | c-Pr | c-Bu |
| 9-85 | c-Pr | 0 | CH₂c-Pr | c-Bu |
| 9-86 | c-Pr | 1 | CH₂c-Pr | c-Bu |
| 9-87 | c-Pr | 2 | CH₂c-Pr | c-Bu |
| 9-88 | c-Pr | 0 | CH₂CH₂OMe | c-Bu |
| 9-89 | c-Pr | 1 | CH₂CH₂OMe | c-Bu |
| 9-90 | c-Pr | 2 | CH₂CH₂OMe | c-Bu |

**Tabelle 10: Erfindungsgemäße Benzoesäurechloride der Formel (III)**

| | | | | |
|---|---|---|---|---|
| 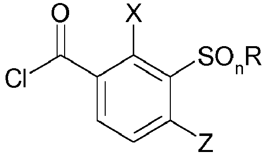 | | | | |

| Nr. | X | n | R | Z |
|---|---|---|---|---|
| 10-1 | Me | 0 | Me | c-Pr |
| 10-2 | Me | 2 | Me | c-Pr |
| 10-3 | Me | 0 | Et | c-Pr |
| 10-4 | Me | 2 | Et | c-Pr |
| 10-5 | Me | 0 | c-Pr | c-Pr |
| 10-6 | Me | 2 | c-Pr | c-Pr |
| 10-7 | Me | 0 | CH₂c-Pr | c-Pr |
| 10-8 | Me | 2 | CH₂c-Pr | c-Pr |
| 10-9 | Me | 0 | CH₂CH₂OMe | c-Pr |
| 10-10 | Me | 2 | CH₂CH₂OMe | c-Pr |
| 10-11 | Me | 0 | Me | c-Bu |
| 10-12 | Me | 2 | Me | c-Bu |
| 10-13 | Me | 0 | Et | c-Bu |
| 10-14 | Me | 2 | Et | c-Bu |
| 10-15 | Me | 0 | c-Pr | c-Bu |
| 10-16 | Me | 2 | c-Pr | c-Bu |
| 10-17 | Me | 0 | CH₂c-Pr | c-Bu |
| 10-18 | Me | 2 | CH₂c-Pr | c-Bu |
| 10-19 | Me | 0 | CH₂CH₂OMe | c-Bu |
| 10-20 | Me | 2 | CH₂CH₂OMe | c-Bu |
| 10-21 | Et | 0 | Me | c-Pr |
| 10-22 | Et | 2 | Me | c-Pr |
| 10-23 | Et | 0 | Et | c-Pr |
| 10-24 | Et | 2 | Et | c-Pr |
| 10-25 | Et | 0 | c-Pr | c-Pr |
| 10-26 | Et | 2 | c-Pr | c-Pr |
| 10-27 | Et | 0 | CH₂c-Pr | c-Pr |
| 10-28 | Et | 2 | CH₂c-Pr | c-Pr |
| 10-29 | Et | 0 | CH₂CH₂OMe | c-Pr |
| 10-30 | Et | 2 | CH₂CH₂OMe | c-Pr |
| 10-31 | Et | 0 | Me | c-Bu |
| 10-32 | Et | 2 | Me | c-Bu |
| 10-33 | Et | 0 | Et | c-Bu |
| 10-34 | Et | 2 | Et | c-Bu |
| 10-35 | Et | 0 | c-Pr | c-Bu |
| 10-36 | Et | 2 | c-Pr | c-Bu |
| 10-37 | Et | 0 | CH₂c-Pr | c-Bu |
| 10-38 | Et | 2 | CH₂c-Pr | c-Bu |
| 10-39 | Et | 0 | CH₂CH₂OMe | c-Bu |
| 10-40 | Et | 2 | CH₂CH₂OMe | c-Bu |
| 10-41 | c-Pr | 0 | Me | c-Pr |
| 10-42 | c-Pr | 2 | Me | c-Pr |
| 10-43 | c-Pr | 0 | Et | c-Pr |
| 10-44 | c-Pr | 2 | Et | c-Pr |
| 10-45 | c-Pr | 0 | c-Pr | c-Pr |
| 10-46 | c-Pr | 2 | c-Pr | c-Pr |
| 10-47 | c-Pr | 0 | CH₂c-Pr | c-Pr |
| 10-48 | c-Pr | 2 | CH₂c-Pr | c-Pr |
| 10-49 | c-Pr | 0 | CH₂CH₂OMe | c-Pr |
| 10-50 | c-Pr | 2 | CH₂CH₂OMe | c-Pr |
| 10-51 | c-Pr | 0 | Me | c-Bu |
| 10-52 | c-Pr | 2 | Me | c-Bu |
| 10-53 | c-Pr | 0 | Et | c-Bu |
| 10-54 | c-Pr | 2 | Et | c-Bu |
| 10-55 | c-Pr | 0 | c-Pr | c-Bu |
| 10-56 | c-Pr | 2 | c-Pr | c-Bu |
| 10-57 | c-Pr | 0 | CH₂c-Pr | c-Bu |
| 10-58 | c-Pr | 2 | CH₂c-Pr | c-Bu |
| 10-59 | c-Pr | 0 | CH₂CH₂OMe | c-Bu |
| 10-60 | c-Pr | 2 | CH₂CH₂OMe | c-Bu |

Zu zahlreichen in obigen Tabellen genannten erfindungsgemäßen Verbindungen der Formeln (I) und (II) werden nachfolgend NMR-Daten im sogenannten NMR-Peak-Listenverfahren offenbart. Dabei werden die ¹H-NMR-Daten ausgewählter Beispiele werden in Form von ¹H-NMR-Peaklisten notiert. Zu jedem Signalpeak wird erst der δ-Wert in ppm und dann die Signalintensität in runden Klammern aufgeführt. Die δ-Wert - Signalintensitäts- Zahlenpaare von verschiedenen Signalpeaks werden durch Semikolons voneinander getrennt aufgelistet. Die Peakliste eines Beispieles hat daher die Form:
δ₁ (Intensität₁⁾; δ₂ (Intensität₂);........; δᵢ (Intensitätᵢ⁾;......; δ_{η} (Intensitätₙ)
Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden. Die Listen der ¹H-NMR-Peaks sind ähnlich den klassischen ¹H-NMR-Ausdrucken und enthalten somit gewöhnlich alle Peaks, die bei einer klassischen NMR-Interpretation aufgeführt werden. Darüber hinaus können sie wie klassische ¹H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im Delta-Bereich von Lösungsmitteln und/oder Wasser sind in unseren Listen von 1H-NMR-Peaks die gewöhnlichen Lösungsmittelpeaks, zum Beispiel Peaks von DMSO in DMSO-D₆ und der Peak von Wasser, gezeigt, die gewöhnlich im Durchschnitt eine hohe Intensität aufweisen.

Die Peaks von Stereoisomeren der erfindungsgemäßen Verbindungen und/oder Peaks von Verunreinigungen haben gewöhnlich im Durchschnitt eine geringere Intensität als die Peaks der erfindungsgemäßen Verbindungen (zum Beispiel mit einer Reinheit von >90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der erfindungsgemäßen Verbindungen isolieren, wobei gegebenenfalls zusätzliche Intensitätsfilter eingesetzt werden. Diese Isolierung wäre ähnlich dem betreffenden Peak-Picking bei der klassischen ¹H-NMR-Interpretation.

**Verbindungen der Formel (I):**

| |
|---|
| Beispiel 1-1: ¹H-NMR(400,0 MHz, CDCl₃): δ= 7,556(0,8);7,535(0,8);7,519(0,6);7,2694(0,6);7,2685(0,6);7,268(0, 8);7,267(1,0);7,266(1,1);7,260(117,2);6,996(0,6);6,809(0,9);6,789(0,9);4,101(11,9);2,840(0,8);2,789(7,6);2,307( 16,0);2,267(0,7);1,547(4,4);1,143(1,2);1,139(1,1);1,134(0,6);1,127(0,7);1,122(1,1);1,117(1,2);1,106(0,6);0,786(0 ,5);0,775(1,2);0,770(1,2);0,762(1,1);0,757(1,3);0,008(1,5);0,000(54,5);-0,009(1,5) |
| Beispiel 1-2: ¹H-NMR(400,0 MHz, CDCl₃): δ= 9,854(0,6);7,605(1,3);7,584(1,3);7,519(1,8);7,260(323,9);6,996(1, 7);6,933(1,5);6,912(1,4);4,103(0,6);4,088(14,6);3,022(16,0);2,831(0,7);2,708(8,1);2,307(0,6);2,096(3,3);1,545(1 6,5);1,254(0,5);1,195(0,6);1,177(1,1);1,156(1,2);0,881(0,6);0,867(0,6);0,858(0,6);0,805(0,7);0,796(0,6);0,008(4, 6);0,000(137,8);-0,009(3,9);-0,150(0,5) |
| Beispiel 1-3: ¹H-NMR(400,0 MHz, CDCl₃): δ= 7,704(0,8);7,683(0,9);7,262(29,8);7,093(1,1);7,073(1,0);4,115(8,3) ;3,264(16,0);3,034(0,8);3,028(0,6);2,823(8,5);1,285(0,7);1,256(3,3);1,215(0,7);1,203(1,6);1,199(1,6);1,194(0,9); 1,187(0,9);1,182(1,5);1,178(1,5);1,166(0,6);0,880(0,8);0,874(0,9);0,862(1,9);0,859(2,0);0,849(1,8);0,845(1,8);0, 833(0,7);0,000(13,3) |
| Beispiel 2-1: ¹H-NMR(400,0 MHz, CDCl₃): δ= 7,542(0,6);7,519(1,3);7,260(142,8);6,996(0,8);6,800(0,8);6,780(0, 8);4,486(1,0);4,468(3,1);4,450(3,1);4,432(1,0);2,850(0,5);2,836(1,1);2,829(0,9);2,823(0,7);2,815(0,6);2,785(9,1); 2,310(1,3);2,304(16,0);2,264(1,6);2,044(0,7);1,637(3,3);1,619(6,9);1,600(3,4);1,591(0,6);1,573(1,4);1,555(2,1);1 ,259(0,6);1,151(0,6);1,140(1,6);1,135(1,7);1,130(0,9);1,124(0,9);1,119(1,7);1,114(1,8);1,103(0,7);0,882(0,6);0,7 82(0,7);0,770(1,8);0,766(1,9);0,757(1,8);0,753(2,1);0,741(0,7);0,008(1,6);0,000(50,7);-0,009(1,5) |
| Beispiel 2-2: ¹H-NMR(400,0 MHz, CDCl₃): δ= 7,605(1,1);7,585(1,2);7,519(1,2);7,374(0,5);7,260(199,6);7,248(0, 6);6,996(1,2);6,921(1,5);6,900(1,4);4,454(0,8);4,437(2,1);4,435(2,2);4,419(2,2);4,417(2,2);4,401(0,8);3,014(16,0 );2,782(0,8);2,690(7,5);2,306(1,5);2,090(0,8);1,637(0,6);1,631(3,9);1,618(1,2);1,612(8,6);1,594(4,1);1,551(3,2);1 ,177(1,4);1,171(0,9);1,161(0,9);1,156(1,4);1,140(0,5);0,878(0,5);0,864(0,6);0,855(0,6);0,794(0,6);0,786(0,6);0,7 71(0,5);0,008(2,4);0,000(71,6);-0,009(2,1) |
| Beispiel 2-3: ¹H-NMR(400,0 MHz, CDCl₃): δ= 7,687(1,0);7,666(1,0);7,271(0,6);7,270(0,7);7,269(0,8);7,268(0,9); 7,261(83,2);7,095(1,2);7,074(1,1);4,501(0,9);4,482(2,8);4,464(2,8);4,446(0,9);3,264(16,0);3,043(0,7);2,834(9,0); 2,006(1,2);1,642(3,4);1,624(7,6);1,606(3,5);1,574(1,0);1,219(0,5);1,207(1,3);1,202(1,3);1,198(0,7);1,191(0,7);1, 185(1,2);1,181(1,3);1,170(0,6);0,873(0,6);0,862(1,3);0,858(1,4);0,848(1,3);0,844(1,4);0,008(0,9);0,000(27,8);-0,009(0,8) |
| Beispiel 3-1: ¹H-NMR(400,0 MHz, CDCl₃): δ= 7,519(1,3);7,495(0,7);7,2763(0,5);7,2755(0,6);7,275(0,6);7,273(0, 7);7,2723(0,8);7,2715(0,9);7,271(1,0);7,270(1,1);7,2683(1,5);7,2675(1,7);7,267(1,9);7,260(158,8);6,996(0,9);6,8 00(0,9);6,780(0,9);5,300(4,4);4,412(1,4);4,394(1,8);4,375(1,5);2,837(0,7);2,781(7,0);2,314(0,6);2,305(16,0);2,04 9(0,8);2,031(1,5);2,012(1,5);1,994(0,9);1,551(2,0);1,153(0,5);1,142(1,3);1,137(1,3);1,132(0,7);1,126(0,7);1,121( 1,3);1,116(1,3);1,105(0,6);1,003(3,1);0,984(6,6);0,966(3,0);0,780(0,6);0,769(1,3);0,764(1,4);0,755(1,3);0,751(1, 5);0,739(0,5);0,008(2,0);0,006(0,8);0,000(60,1);-0,006(0,7);-0,007(0,5);-0,009(1,7) |
| Beispiel 3-2: ¹H-NMR(400,0 MHz, CDCl₃): δ= 7,584(1,0);7,564(1,0);7,519(2,2);7,292(0,6);7,276(1,4);7,273(2,0); 7,260(387,6);7,252(1,5);7,250(0,9);7,249(0,9);7,210(0,7);6,996(2,2);6,927(1,5);6,907(1,4);4,384(1,1);4,381(1,2); 4,366(2,0);4,361(1,9);4,347(1,2);4,344(1,2);3,247(1,2);3,016(16,0);2,834(0,7);2,702(7,1);2,093(0,9);2,044(1,1);2 ,025(2,1);2,007(2,1);1,988(1,2);1,548(17,9);1,189(0,5);1,174(1,4);1,168(0,9);1,153(1,3);1,007(4,1);0,995(0,9);0, 989(8,7);0,970(3,8);0,860(0,6);0,851(0,6);0,799(0,7);0,791(0,6);0,008(4,3);0,000(134,5);-0,009(3,9) |
| Beispiel 3-3: ¹H-NMR(400,0 MHz, CDCl₃): δ= 7,664(1,0);7,644(1,1);7,519(0,7);7,311(0,8);7,273(1,0);7,272(0,9); 7,261(126,8);7,237(1,0);7,211(0,6);7,183(1,0);7,176(0,5);7,167(0,6);7,165(0,7);7,163(0,7);7,162(0,6);7,092(1,2); 7,071(1,2);6,997(0,8);4,427(1,5);4,409(1,9);4,391(1,5);3,356(0,6);3,270(0,8);3,261(16,0);3,235(0,5);3,043(0,8);2 ,836(9,4);2,356(3,7);2,088(1,2);2,050(0,9);2,032(1,6);2,013(1,7);1,995(1,0);1,567(1,1);1,219(0,5);1,207(1,3);1,2 03(1,4);1,198(0,8);1,191(0,8);1,186(1,3);1,182(1,4);1,170(0,6);1,010(3,5);0,991(7,4);0,973(3,3);0,869(0,6);0,857 (1,4);0,854(1,6);0,844(1,4);0,840(1,5);0,828(0,6);0,008(1,4);0,000(45,9);-0,009(1,4) |
| Beispiel 4-1: ¹H-NMR(400,0 MHz, CDCl₃): δ= 7,519(1,2);7,494(1,8);7,456(0,9);7,436(0,9);7,260(214,1);6,996(1, 1);6,777(1,0);6,756(1,0);3,880(10,0);2,830(0,7);2,757(8,3);2,297(16,0);2,045(0,6);1,542(13,1);1,146(0,5);1,134( 1,2);1,130(1,2);1,125(0,6);1,118(0,7);1,113(1,2);1,108(1,1);1,097(0,5);0,768(0,5);0,757(1,2);0,752(1,2);0,743(1, 1);0,739(1,3);0,008(2,8);0,000(95,1);-0,009(2,6) |
| Beispiel 4-2: ¹H-NMR(400,0 MHz, d₆-DMSO):δ= 10,987(1,4);7,877(2,3);7,562(0,7);7,543(0,8);7,048(1,1);7,028( 1,1);3,743(16,0);3,420(0,5);3,390(0,5);3,359(0,7);3,354(0,9);3,350(1,0);3,320(733,1);3,297(31,4);3,270(1,4);3,1 74(0,7);3,161(0,7);3,018(14,4);2,863(0,5);2,844(0,6);2,675(1,3);2,670(1,8);2,665(1,4);2,661(0,8);2,646(9,4);2,54 0(1,1);2,523(4,9);2,519(7,6);2,510(96,8);2,506(208,5);2,501(288,8);2,496(201,8);2,492(89,9);2,474(1,2);2,469(1 ,1);2,460(0,8);2,456(0,8);2,451(0,9);2,447(0,5);2,337(0,5);2,332(1,2);2,328(1,7);2,323(1,3);2,299(0,5);1,094(0,5) ;1,085(0,6);0,999(0,5);0,926(0,6);0,913(0,7);0,735(0,6);0,720(0,6);0,000(0,5) |
| Beispiel 4-3: ¹H-NMR(400,0 MHz, CDCl₃): δ= 7,604(0,8);7,584(0,9);7,410(1,1);7,261(52,3);7,063(1,1);7,042(1,1) ;3,890(9,3);3,230(16,0);3,042(0,7);2,815(9,1);2,055(0,9);1,285(0,6);1,255(1,8);1,212(0,6);1,200(1,4);1,196(1,4); 1,191(0,8);1,184(0,8);1,179(1,4);1,175(1,4);1,163(0,7);0,849(0,7);0,837(1,5);0,834(1,6);0,824(1,4);0,820(1,5);0, 808(0,5);0,008(0,7);0,000(23,5);-0,009(0,7) |
| Beispiel 7-1: ¹H-NMR(400,0 MHz, CDCl₃): δ= 7,261(70,3);6,751(1,2);6,731(1,2);5,300(1,6);2,821(0,9);2,762(11, 2);2,543(10,9);2,296(16,0);2,007(7,2);1,561(2,5);1,133(0,6);1,121(1,4);1,117(1,4);1,112(0,8);1,105(0,8);1,100(1, 4); 1,095(1,4); 1,084(0,6);0,759(0,7);0,748(1,6);0,744(1,6);0,735(1,5);0,731(1,6);0,719(0,5);0,008(0,9);0,000(28,7 );-0,009(0,7) |
| Beispiel 7-2: ¹H-NMR(400,0 MHz, CDCl₃): δ= 7,532(0,7);7,520(0,7);7,511(0,8);7,261(98,5);7,228(0,7);6,997(0,5) ;6,871(1,5);6,851(1,4);3,001(16,0);2,655(7,4);2,547(15,5);1,333(0,7);1,285(1,0);1,255(0,7);1,119(0,6);1,110(0,7) ;1,106(0,6);1,097(0,8);1,087(0,5);1,076(0,5);0,834(0,6);0,768(0,5);0,758(0,6);0,744(0,5);0,008(1,0);0,000(34,6);-0,009(1,0) |
| Beispiel 7-3: ¹H-NMR(400,0 MHz, d₆-DMSO): δ= 12,049(4,0);7,606(13,5);7,586(15,1);7,145(13,9);7,124(12,9);5, 755(10,3);3,352(147,7);3,314(712,8);3,290(37,0);2,894(7,6);2,880(4,2);2,674(6,3);2,670(9,0);2,665(6,6);2,638(9 6,5);2,539(10,8);2,534(12,5);2,530(10,5);2,523(23,2);2,518(34,1);2,510(496,8);2,505(1075,7);2,501(1494,7);2,4 96(1040,4);2,491(467,1);2,475(130,1);2,332(5,6);2,328(8,5);2,323(6,0);2,073(13,1);1,335(2,9);1,298(3,0);1,259( 4,6);1,250(4,7);1,235(4,9);1,118(4,6);1,107(12,3);1,102(13,3);1,091(7,4);1,086(13,0);1,081(13,4);1,070(5,8);0,8 64(5,7);0,848(16,0);0,835(14,5);0,824(4,3);0,034(2,6);0,008(15,2);0,000(516,1);-0,009(15,7) |

**Verbindungen der Formel (II):**

| |
|---|
| Beispiel 9-1: ¹H-NMR (400,0 MHz, d₆-DMSO): δ= 7,628(1,5); 7,608 (1,6); 6,806 (1,3); 6,785 (1,2); 3,320 (3,2); 2,772 (0,6); 2,749 (9,5); 2,737 (0,9); 2,723 (0,5); 2,511 (4,4); 2,507 (9,5); 2,502 (13,3); 2,498 (9,6); 2,493 (4,7); 2,478 (0,6); 2,258 (16,0); 2,029 (0,7); 1,078 (1,2); 1,072 (1,3); 1,068 (0,7); 1,062 (0,7); 1,057 (1,3); 1,051 (1,3); 1,041 (0,6); 0,752 (0,5); 0,741 (1,3); 0,736 (1,4); 0,728 (1,3); 0,723 (1,4); 0,712 (0,5); 0,000 (5,4) |

### B. Formulierungsbeispiele

a) Ein Stäubemittel wird erhalten, indem man 10 Gew.-Teile einer Verbindung der Formel (I) und/oder deren Salze und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I) und/oder deren Salze, 64 Gew.-Teile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gew.-Teile einer Verbindung der Formel (I) und/oder deren Salze mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277 C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I) und/oder deren Salze, 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man
   75 Gew.-Teile einer Verbindung der Formel (I) und/oder deren Salze,
   10 Gew.-Teile ligninsulfonsaures Calcium,
   5 Gew.-Teile Natriumlaurylsulfat,
   3 Gew.-Teile Polyvinylalkohol und
   7 Gew.-Teile Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man
   25 Gew.-Teile einer Verbindung der Formel (I) und/oder deren Salze,
   5 Gew.-Teile 2,2'-dinaphthylmethan-6,6'-disulfonsaures Natrium
   2 Gew.-Teile oleoylmethyltaurinsaures Natrium,
   1 Gew.-Teil Polyvinylalkohol,
   17 Gew.-Teile Calciumcarbonat und
   50 Gew.-Teile Wasser
auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

Die hier verwendeten Abkürzungen bedeuten:

| | | | |
|---|---|---|---|
| ABUTH | Abutilon theophrasti | ALOMY | Alopecurus myosuroides |
| AMARE | Amaranthus retroflexus | AVEFA | Avena fatua |
| CYPES | Cyperus serotinus | ECHCG | Echinochloa crus galli |
| LOLMU | Lolium multiflorum | MATIN | Matricaria inodora |
| PHBPU | Pharbitis purpureum | PHBPU | Pharbitis purpureum |
| POLCO | Polygonum convolvulus | STEME | Stellaria media |
| VERPE | Veronica persica | | |

### 1. Herbizide Wirkung gegen Schadpflanzen im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) oder als Emulsionskonzentrate (EC) formulierten erfindungsgemäßen und zu Vergleichszwecken aus dem Stand der Technik bekannten Verbindungen werden dann als wäßrige Suspension bzw. Emulsion mit einer Wasseraufwandmenge von umgerechnet 600 bis 800 I/ha unter Zusatz von 0,2% Netzmittel auf die Oberfläche der Abdeckerde appliziert. Nach der Behandlung werden die Töpfe im Gewächshaus aufgestellt und unter guten Wachstumsbedingungen für die Testpflanzen gehalten. Die visuelle Bonitur der Schäden an den Versuchspflanzen erfolgt nach einer Versuchszeit von 3 Wochen im Vergleich zu unbehandelten Kontrollen (herbizide Wirkung in Prozent (%): 100% Wirkung = Pflanzen sind abgestorben, 0 % Wirkung = wie Kontrollpflanzen).

Die Versuche zeigen, dass die geprüften erfindungsgemäßen Verbindungen gegen zahlreiche Schadpflanzen eine höhere herbizide Wirkung und geringere Schäden in Kulturpflanzen aufweisen als die strukturell ähnlichsten aus dem Stand der Technik bekannten Verbindungen. Die Daten dieser Versuche sind in den nachfolgenden Tabellen aufgeführt.

**Tabelle A**

| Verbindung | Dosierung [g/ha] | Wirkung gegen | |
|---|---|---|---|
| | | CYPES | POLCO |
| 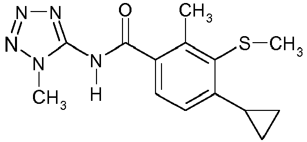 | 320 | 90% | 90% |
| erfindungsgemäße Verbindung Nr. 1-1 | | | |
| 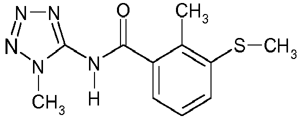 | 320 | 30% | 0% |
| aus WO 2012/028579 A1 bekannte Verbindung Nr. 4-78 | | | |
| 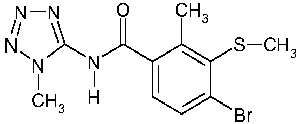 | 320 | 0% | 70% |
| aus WO 2012/028579 A1 bekannte Verbindung Nr. 4-122 | | | |

**Tabelle B**

| Verbindung | Dosierung [g/ha] | Wirkung gegen | |
|---|---|---|---|
| | | CYPES | POLCO |
| 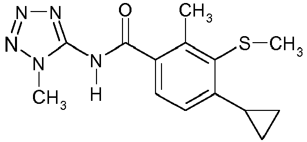 | 320 | 90% | 90% |
| erfindungsgemäße Verbindung Nr. 1-1 | | | |
| 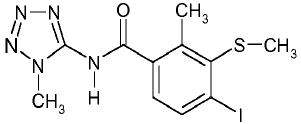 | 320 | 0% | 20% |
| aus WO 2012/028579 A1 bekannte Verbindung Nr. 4-128 | | | |
| 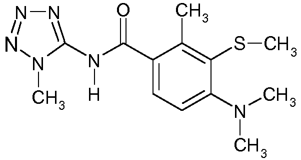 | 320 | 30% | 0% |
| aus WO 2012/028579 A1 bekannte Verbindung Nr. 4-429 | | | |
| 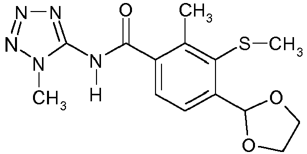 | 320 | 0% | 30% |
| aus WO 2012/028579 A1 bekannte Verbindung | | | |
| 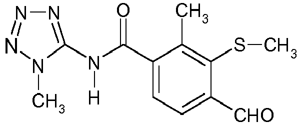 | 320 | 0% | 0% |
| aus WO 2012/028579 A1 bekannte Verbindung | | | |

**Tabelle C**

| Verbindung | Dosierung [g/ha] | Wirkung gegen | |
|---|---|---|---|
| | | ALOMY | AMARE |
| 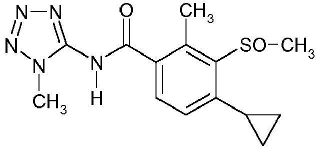 | 320 | 90% | 100% |
| erfindungsgemäße Verbindung Nr. 1-2 | | | |
| 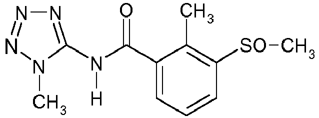 | 320 | 20% | 20% |
| aus WO 2012/028579 A1 bekannte Verbindung Nr. 4-79 | | | |
| 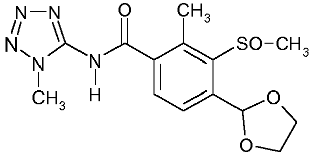 | 320 | 0% | 0% |
| aus WO 2012/028579 A1 bekannte Verbindung | | | |
| 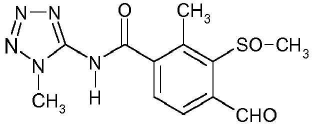 | 320 | 0% | 0% |
| aus WO 2012/028579 A1 bekannte Verbindung | | | |

**Tabelle D**

| Verbindung | Dosierung [g/ha] | Wirkung gegen | |
|---|---|---|---|
| | | ALOMY | POLCO |
| 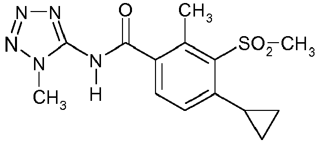 | 320 | 100% | 90% |
| erfindungsgemäße Verbindung Nr. 1-3 | | | |
| 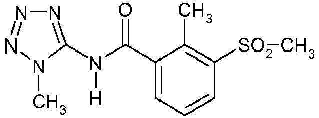 | 320 | 70% | 40% |
| aus WO 2012/028579 A1 bekannte Verbindung Nr. 4-79 | | | |
| 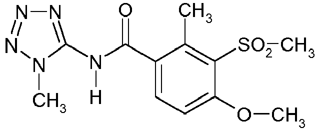 | 320 | 20% | 60% |
| aus WO 2012/028579 A1 bekannte Verbindung Nr. 4-439 | | | |
| 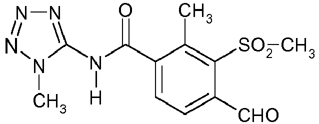 | 320 | 0% | 60% |
| aus WO 2012/028579 A1 bekannte Verbindung | | | |

**Tabelle E**

| Verbindung | Dosierung [g/ha] | Wirkung gegen | |
|---|---|---|---|
| | | AMARE | VERPE |
| 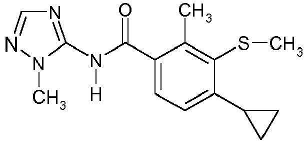 | 320 | 100% | 90% |
| erfindungsgemäße Verbindung Nr. 4-1 | | | |
| 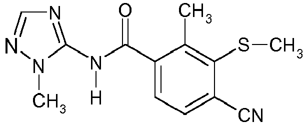 | 320 | 80% | 70% |
| aus WO 2012/028579 A1 bekannte Verbindung Nr. 1-387 | | | |
| 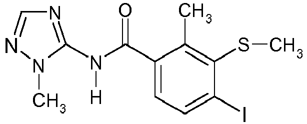 | 320 | 60% | 0% |
| aus WO 2012/028579 A1 bekannte Verbindung Nr. 1-139 | | | |
| 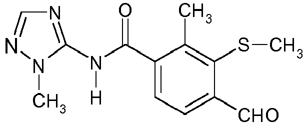 | 320 | 30% | 0% |
| aus WO 2012/028579 A1 bekannte Verbindung | | | |
| 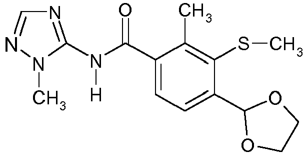 | 320 | 0% | 0% |
| aus WO 2012/028579 A1 bekannte Verbindung | | | |

**Tabelle F**

| Verbindung | Dosierung [g/ha] | Wirkung gegen | |
|---|---|---|---|
| | | AMARE | MATIN |
| 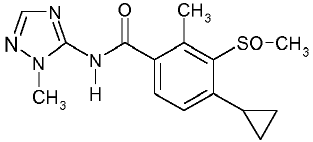 | 320 | 90% | 80% |
| erfindungsgemäße Verbindung Nr. 4-2 | | | |
| 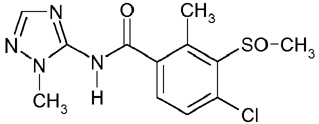 | 320 | 70% | 30% |
| aus WO 2012/028579 A1 bekannte Verbindung Nr. 1-120 | | | |
| 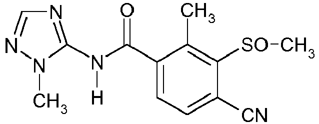 | 320 | 60% | 0% |
| aus WO 2012/028579 A1 bekannte Verbindung Nr. 1-388 | | | |
| 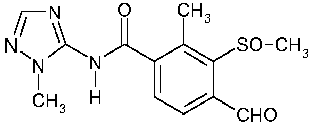 | 320 | 40% | 0% |
| aus WO 2012/028579 A1 bekannte Verbindung | | | |
| 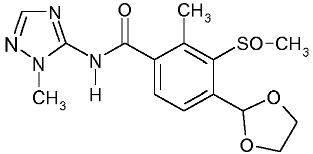 | 320 | 20% | 0% |
| aus WO 2012/028579 A1 bekannte Verbindung | | | |

**Tabelle G**

| Verbindung | Dosierung [g/ha] | Wirkung gegen | |
|---|---|---|---|
| | | ECHCG | AMARE |
| 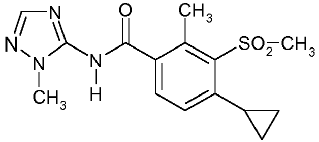 | 320 | 90% | 100% |
| erfindungsgemäße Verbindung Nr. 4-3 | | | |
| 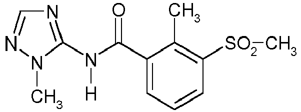 | 320 | 30% | 10% |
| aus WO 2012/028579 A1 bekannte Verbindung Nr. 1-90 | | | |
| 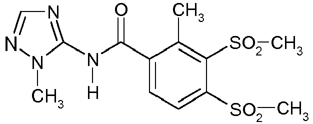 | 320 | 0% | 40% |
| aus WO 2012/028579 A1 bekannte Verbindung Nr. 1-188 | | | |
| 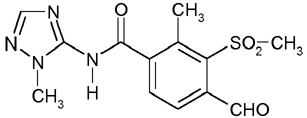 | 320 | 20% | 0% |
| aus WO 2012/028579 A1 bekannte Verbindung | | | |
| 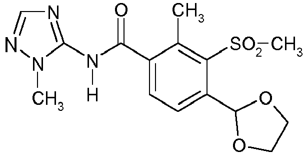 | 320 | 40% | 60% |
| aus WO 2012/028579 A1 bekannte Verbindung | | | |

**Tabelle H**

| Verbindung | Dosierung [g/ha] | Wirkung gegen | |
|---|---|---|---|
| | | AVEFA | LOLMU |
| 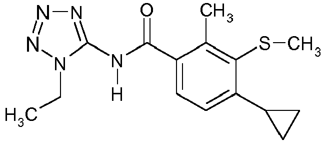 | 320 | 100% | 90% |
| erfindungsgemäße Verbindung Nr. 2-1 | | | |
| 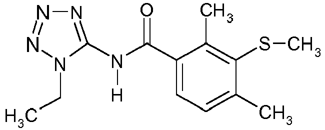 | 320 | 60% | 30% |
| aus WO 2012/028579 A1 bekannte Verbindung | | | |

**Tabelle I**

| Verbindung | Dosierung [g/ha] | Wirkung gegen | |
|---|---|---|---|
| | | LOLMU | PHBPU |
| 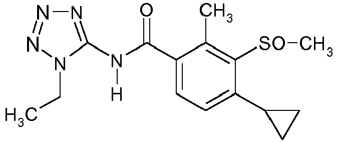 | 320 | 100% | 100% |
| erfindungsgemäße Verbindung Nr. 2-2 | | | |
| 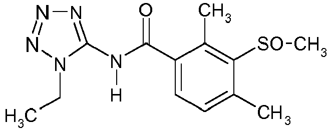 | 320 | 30% | 60% |
| aus WO 2012/028579 A1 bekannte Verbindung Nr. 5-391 | | | |

**Tabelle J**

| Verbindung | Dosierung [g/ha] | Wirkung gegen | |
|---|---|---|---|
| | | CYPES | ABUTH |
| 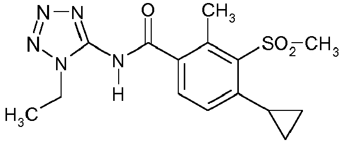 | 80 | 80% | 100% |
| erfindungsgemäße Verbindung Nr. 2-3 | | | |
| 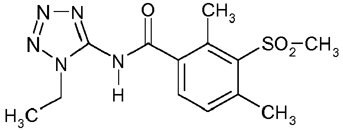 | 80 | 40% | 80% |
| aus WO 2012/028579 A1 bekannte Verbindung | | | |

### 2. Herbizide Wirkung gegen Schadpflanzen im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 I/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen). Die Versuche zeigen, dass die geprüften erfindungsgemäßen Verbindungen gegen zahlreiche Schadpflanzen eine höhere herbizide Wirkung und geringere Schäden in Kulturpflanzen aufweisen als die strukturell ähnlichsten aus dem Stand der Technik bekannten Verbindungen. Die Daten dieser Versuche sind in den nachfolgenden Tabellen aufgeführt.

**Tabelle K**

| Verbindung | Dosierung [g/ha] | Wirkung gegen | |
|---|---|---|---|
| | | MATIN | STEME |
| 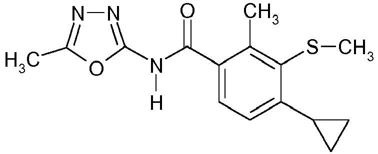 | 80 | 80% | 90% |
| erfindungsgemäße Verbindung Nr. 7-1 | | | |
| 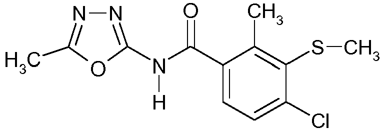 | 80 | 60% | 70% |
| aus WO 2012/26932 A1 bekannte Verbindung Nr. 2-116 | | | |
| 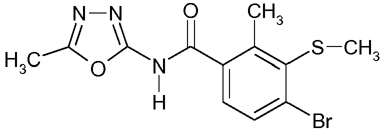 | 80 | 40% | 60% |
| aus WO 2012/26932 A1 bekannte Verbindung Nr. 2-130 | | | |

**Tabelle L**

| Verbindung | Dosierung [g/ha] | Wirkung gegen | |
|---|---|---|---|
| | | MATIN | POLCO |
| 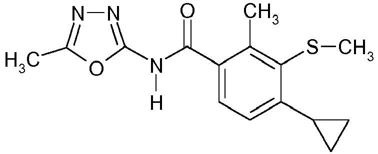 | 80 | 80% | 60% |
| erfindungsgemäße Verbindung Nr. 7-1 | | | |
| 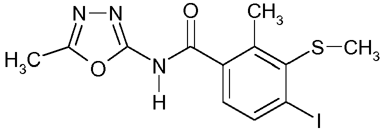 | 80 | 60% | 20% |
| aus WO 2012/26932 A1 bekannte Verbindung Nr. 2-136 | | | |
| 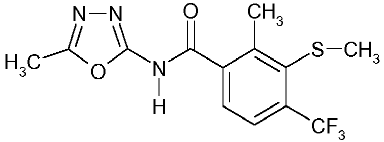 | 80 | 60% | 0% |
| aus WO 2012/26932 A1 bekannte Verbindung Nr. 2-143 | | | |

## Patentansprüche

1. Benzoesäureamide der Formel (I) oder deren Salze 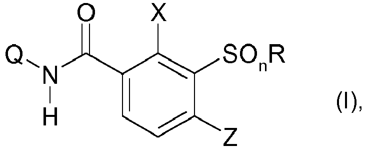 worin die Symbole und Indizes folgende Bedeutungen haben:
Q bedeutet einen Rest Q1, Q2, Q3 oder Q4, 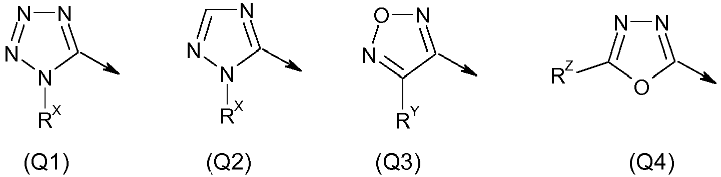
X bedeutet (C₁-C₆)-Alkyl oder (C₃-C₆)-Cycloalkyl,
Z bedeutet (C₃-C₆)-Cycloalkyl,
R bedeutet (C₁-C₆)-Alkyl, (C₃-C₆)-Cycloalkyl, (C₃-C₆)-Cycloalkyl-(C₁-C₆)-alkyl, (C₁-C6)-Alkyl-O-(C1-C6)-alkyl,
R^{X} bedeutet Methyl, Ethyl, n-Propyl, Prop-2-en-1-yl, Methoxyethyl, Ethoxyethyl oder Methoxyethoxyethyl,
R^{Y} bedeutet Methyl, Ethyl, n-Propyl, Chlor oder Amino,
R^{Z} bedeutet Methyl, Ethyl, n-Propyl oder Methoxymethyl,
n bedeutet 0, 1 oder 2.

2. Benzoesäureamide nach Anspruch 1, worin
Q bedeutet einen Rest Q1, Q2, Q3 oder Q4, 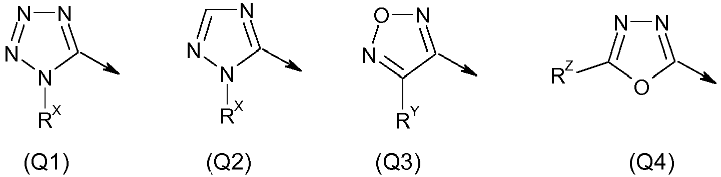
X bedeutet Methyl, Ethyl oder Cyclopropyl,
Z bedeutet Cyclopropyl oder Cyclobutyl,
R bedeutet Methyl, Ethyl, Cyclopropyl, Cyclopropylmethyl oder Methoxyethyl,
R^{X} bedeutet Methyl, Ethyl, n-Propyl, Prop-2-en-1-yl, Methoxyethyl, Ethoxyethyl oder Methoxyethoxyethyl,
R^{Y} bedeutet Methyl, Ethyl, n-Propyl, Chlor oder Amino,
R^{Z} bedeutet Methyl, Ethyl, n-Propyl oder Methoxymethyl,
n bedeutet 0, 1 oder 2.

3. Herbizide Mittel, **gekennzeichnet durch** einen herbizid wirksamen Gehalt an mindestens einer Verbindung der Formel (I) gemäß einem der Ansprüche 1 oder 2.

4. Herbizide Mittel nach Anspruch 3 in Mischung mit Formulierungshilfsmitteln.

5. Verfahren zur Bekämpfung unerwünschter Pflanzen, **dadurch gekennzeichnet, daß** man eine wirksame Menge mindestens einer Verbindung der Formel (I) gemäß Anspruch 1 oder 2 oder eines herbiziden Mittels gemäß Anspruch 3 oder 4 auf die Pflanzen oder auf den Ort des unerwünschten Pflanzenwachstums appliziert.

6. Verwendung von Verbindungen der Formel (I) gemäß Anspruch 1 oder 2 oder eines herbiziden Mittels gemäß Anspruch 3 oder 4 zur Bekämpfung unerwünschter Pflanzen.

7. Verwendung gemäß Anspruch 6, **dadurch gekennzeichnet, daß** die Verbindungen der Formel (I) zur Bekämpfung unerwünschter Pflanzen in Kulturen von Nutzpflanzen eingesetzt werden.

8. Verwendung gemäß Anspruch 7, **dadurch gekennzeichnet, daß** die Nutzpflanzen transgene Nutzpflanzen sind.

9. Benzoesäuren der Formel (II), 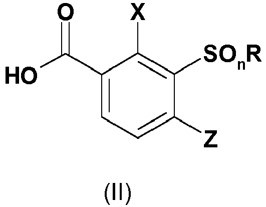 worin X, R, Z und n wie in Anspruch 1 oder 2 definiert sind.

10. Benzoesäurechloride der Formel (III) 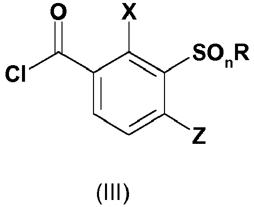 worin X, R, Z und n wie in Anspruch 1 oder 2 definiert sind.

## Claims

1. Benzamides of the formula (I) or salts thereof 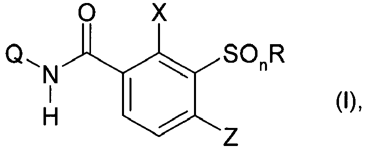 where the symbols and indices are each defined as follows:
Q is a Q1, Q2, Q3 or Q4 radical, 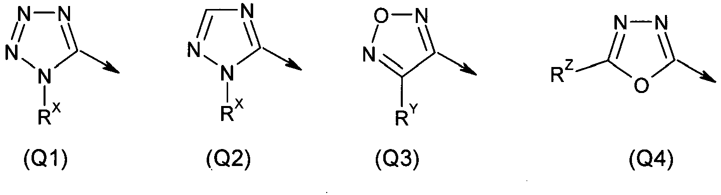
X is (C₁-C₆) -alkyl or (C₃-C₆) -cycloalkyl,
Z is (C₃-C₆) -cycloalkyl,
R is (C₁-C₆) -alkyl, (C₃-C₆) -cycloalkyl, (C₃-C₆)-cycloalkyl- (C₁-C₆) -alkyl, (C₁-C₆) -alkyl-O- (C₁-C₆) -alkyl,
R^{X} is methyl, ethyl, n-propyl, prop-2-en-1-yl, methoxyethyl, ethoxyethyl or methoxyethoxyethyl,
R^{Y} is methyl, ethyl, n-propyl, chlorine or amino,
R^{Z} is methyl, ethyl, n-propyl or methoxymethyl, n is 0, 1 or 2.

2. Benzamides according to Claim 1, in which
Q is a Q1, Q2, Q3 or Q4 radical, 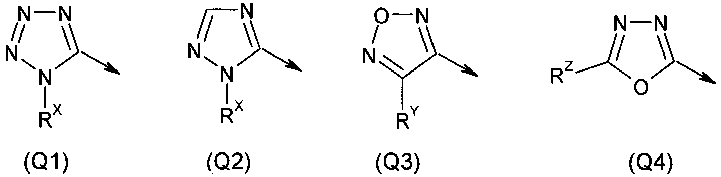
X is methyl, ethyl or cyclopropyl,
Z is cyclopropyl or cyclobutyl,
R is methyl, ethyl, cyclopropyl, cyclopropylmethyl or methoxyethyl,
R^{X} is methyl, ethyl, n-propyl, prop-2-en-1-yl, methoxyethyl, ethoxyethyl or methoxyethoxyethyl,
R^{Y} is methyl, ethyl, n-propyl, chlorine or amino,
R^{Z} is methyl, ethyl, n-propyl or methoxymethyl,
n is 0, 1 or 2.

3. Herbicidal compositions **characterized by** a herbicidally active content of at least one compound of the formula (I) according to either of Claims 1 and 2.

4. Herbicidal compositions according to Claim 3 in a mixture with formulation auxiliaries.

5. Method of controlling unwanted plants, **characterized in that** an effective amount of at least one compound of the formula (I) according to Claim 1 or 2 or of a. herbicidal composition according to Claim 3 or 4 is applied to the plants or to the site of the unwanted vegetation.

6. Use of compounds of the formula (I) according to Claim 1 or 2 or of a herbicidal composition according to Claim 3 or 4 for controlling unwanted plants.

7. Use according to Claim 6, **characterized in that** the compounds of the formula (I) are used for controlling unwanted plants in crops of useful plants.

8. Use according to Claim 7, **characterized in that** the useful plants are transgenic useful plants.

9. Benzoic acids of the formula (II) 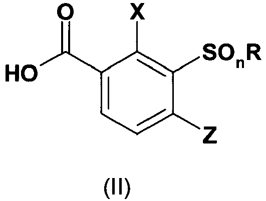 in which X, R, Z and n are as defined in Claim 1 or 2.

10. Benzoyl chlorides of the formula (III) 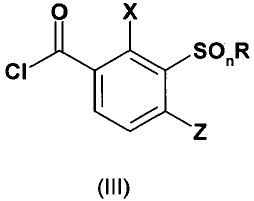 in which X, R, Z and n are as defined in Claim 1 or 2.

## Revendications

1. Amides d'acides benzoïques de formule (I) ou leurs sels 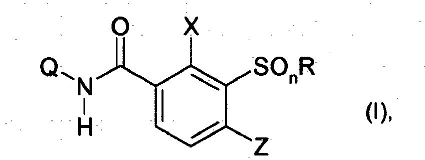 dans laquelle les symboles et les indices ont les significations suivantes :
Q signifie un radical Q1, Q2, Q3 ou Q4, 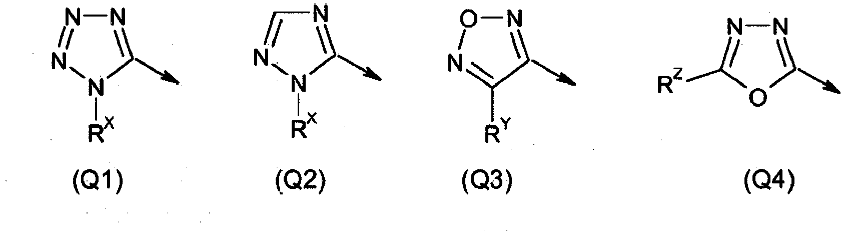
X signifie alkyle en (C₁-C₆) ou cycloalkyle en (C₃-C₆) ,
Z signifie cycloalkyle en (C₃-C₆),
R signifie alkyle en (C₁-C₆), cycloalkyle en (C₃-C₆), cycloalkyle en (C₃-C₆) -alkyle en (C₁-C₆), alkyle en (C₁-C₆)-O-alkyle en (C₁-C₆),
R^{X} signifie méthyle, éthyle, n-propyle, prop-2-én-1-yle, méthoxyéthyle, éthoxyéthyle ou méthoxyéthoxyéthyle,
R^{Y} signifie méthyle, éthyle, n-propyle, chlore ou amino,
R^{Z} signifie méthyle, éthyle, n-propyle ou méthoxyméthyle,
n signifie 0, 1 ou 2.

2. Amides d'acides benzoïques selon la revendication 1, dans lesquels
Q signifie un radical Q1, Q2, Q3 ou Q4, 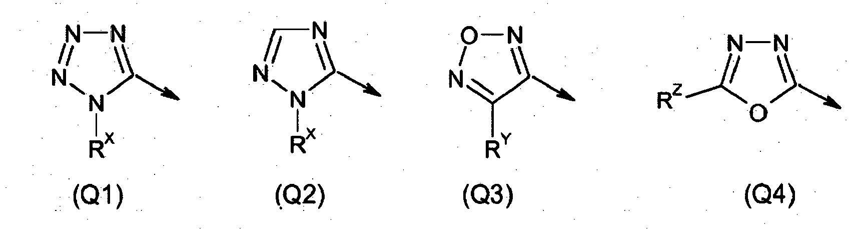
X signifie méthyle, éthyle ou cyclopropyle,
Z signifie cyclopropyle ou cyclobutyle,
R signifie méthyle, éthyle, cyclopropyle, cyclopropylméthyle ou méthoxyéthyle,
R^{X} signifie méthyle, éthyle, n-propyle, prop-2-én-1-yle, méthoxyéthyle, éthoxyéthyle ou méthoxyéthoxyéthyle,
R^{Y} signifie méthyle, éthyle, n-propyle, chlore ou amino,
R^{Z} signifie méthyle, éthyle, n-propyle ou méthoxyméthyle,
n signifie 0, 1 ou 2.

3. Agents herbicides, **caractérisés par** une teneur efficace du point de vue herbicide en au moins un composé de formule (I) selon l'une quelconque des revendications 1 ou 2.

4. Agents herbicides selon la revendication 3 en mélange avec des adjuvants de formulation.

5. Procédé de lutte contre des plantes indésirables, **caractérisé en ce qu'**une quantité efficace d'au moins un composé de formule (I) selon la revendication 1 ou 2 ou d'un agent herbicide selon la revendication 3 ou 4 est appliquée sur les plantes ou à l'emplacement de la végétation indésirable.

6. Utilisation de composés de formule (I) selon la revendication 1 ou 2 ou d'un agent herbicide selon la revendication 3 ou 4 pour lutter contre des plantes indésirables.

7. Utilisation selon la revendication 6, **caractérisée en ce que** les composés de formule (I) sont utilisés pour lutter contre des plantes indésirables dans des cultures de plantes utiles.

8. Utilisation selon la revendication 7, **caractérisée en ce que** les plantes utiles sont des plantes utiles transgéniques.

9. Acides benzoïques de formule (II) 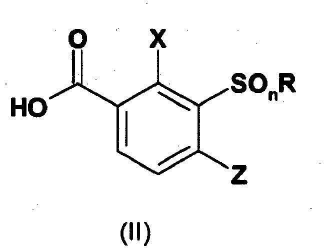 dans laquelle X, R, Z et n sont tels que définis dans la revendication 1 ou 2.

10. Chlorures d'acides benzoïques de formule (III) 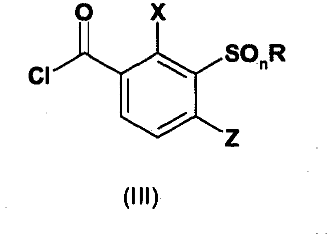 dans laquelle X, R, Z et n sont tels que définis dans la revendication 1 ou 2.
